# NEW EUROPEAN PATENT SPECIFICATION

(11) **EP 0 293 394 B2**
(45) Date of publication and mention of the opposition decision: **29.10.2003**
(45) Mention of the grant of the patent: 20.04.1994
(21) Application number: 87902884.3
(22) Date of filing: 30.01.1987
(51) Int. Cl.: C12N 15/12, C12N 15/52, C12N 9/68, A61K 38/36

(54) **NOVEL THROMBOLYTIC PROTEINS**
NEUE THROMBOLYTISCHE PROTEINE
NOUVELLES PROTEINES THROMBOLYTIQUES

(30) Priority: 31.01.1986 US 825104; 18.04.1986 US 853781; 09.05.1986 US 861699; 03.07.1986 US 882051
(43) Date of publication of application: 07.12.1988
(73) Proprietor: Genetics Institute, LLC, Cambridge, MA 02140 (US)
(72) Inventor: LARSEN, Glenn, R., Sudbury, MA 02176 (US); AHERN, Tim, J., Cambridge, MA 02139 (US)
(74) Representative: VOSSIUS & PARTNER
(86) International application number: US8700257
(87) International publication number: WO87004722

(56) References cited:
- EP-A- 0 042 246
- EP-A- 0 178 105
- EP-A- 0 207 589
- EP-A- 0 238 304
- EP-A- 0 238 304
- EP-A1- 0 093 619
- EP-A1- 0 207 589
- EP-A1- 0 231 624
- EP-A1- 0 241 208
- EP-A1- 0 241 208
- EP-A1- 0 241 208
- EP-A2- 0 178 105
- EP-A2- 0 196 920
- EP-A2- 0 196 920
- EP-A2- 0 199 574
- EP-A2- 0 227 462
- EP-A2- 0 238 304
- EP-A2- 0 277 751
- WO-A-84/01786
- WO-A-84/01786
- WO-A-86/01538
- WO-A-87/05934
- WO-A-89/00197
- DE-A- 3 537 176
- DE-A1- 3 537 176
- DE-A1- 3 537 176
- BIOCHEMISTRY, vol. 26, no. 2, January 1987, Easton, Pennsylvania, USA; K. M. TATE et al, "Functional role of proteolytic cleavage at arginine-275 of human tissue plasminogen activator as assessed by site-directed mutagenesis", pages 338-343
- PROCEEDINGS NATIONAL ACADEMY SCIENCES, vol. 81, September 1984, Washington, D.C., USA; T. NY et al, "The structure of the human tissue-type plasminogen activator gene: Correlation of intron and exon structures of functional and structural domains", pages 5355-5359
- MOLECULAR AND CELLULAR BIOLOGY, vol. 5, no. 7, July 1985, Washington, D.C., USA; R. KAUFMAN et al, "Coamplication and coexpression of human tissue-type plasminogen activator and murine-dihydrofolate reductase sequence of Chinese hamster ovary cells", pages 1750-1759
- FEDERATION OF EUROPEAN BIOCHEMICAL SOCIETIES, vol. 189, no. 1, September 1985, Amsterdam, NL; H. KAGITANI et al, "Expression of E. coli of finger-domain lacking tissue-type plasminogen activator with high fibrin affinity", pages 145-149
- NATURE, vol. 301, January 1983, London, GB; D. PENNICA et al, "Cloning and expression of human tissue-type plasminogen activator cDNA in E. coli", pages 214-221
- FEDERATION OF EUROPEAN BIOCHEMICAL SOCIETIES, vol. 163, no. 1, October 1983, Amsterdam, NL; L. BANYAI et al, "Common evolutionary origin of the fibrin-binding structures of fibronectin and tissue-type plasminogen activator", pages 37-41
- TRENDS IN BIOTECHNOLOGY, vol. 3, no. 2, February 1985, Amsterdam, NL; P. BERMAN et al, "Engineering glycoproteins for use as pharmaceuticals", pages 51-53
- Tate et al., Biochemistry 26, 29, 338 to 343
- Pohl et al., FEBS 168, 29 to 32
- van Zonneveld, PNAS 83, 4670-4674
- Rijken et al., Thromb. Haemo. 0358
- Rijken and Emeis, Biochem., J. 238, 643 to 646
- Kagitani et al., FEBS 189, 145 to 149
- Little et al., Biochemistry 23, 6191 to 6195
- van Zonneveld et al., J Cell. Biochem. 32, 169 to 178
- Declaration of Dr. Fritsch
- Collen et al., Blood 71 (1988), 216-219
- Cambien et al., Cardiovascular Pharmacology 11, (1988), 468-472

## Description

This invention relates to substances having tissue plasminogen activator-type (t-PA) activity. More specifically, this invention relates to "recombinant" thrombolytic proteins, a process for obtaining the proteins from genetically engineered cells, and the therapeutic use of the substances as thrombolytic agents.

These proteins are active thrombolytic agents which, it is contemplated, possess improved fibrinolytic profiles relative to native human t-PA. This may be manifested as increased affinity to fibrin, decreased reactivity with inhibitors of t-PA, faster rate of thrombolysis, increased fibrinolytic activity and/or prolonged biological half-life. It is also contemplated that-proteins of this invention can be more conveniently prepared in more homogeneous form than can native human t-PA. An improved overall pharmacokinetic profile is contemplated for these proteins.

The structure of native human t-PA can be viewed as comprising an amino (N-) terminus of about 91 amino acid residues, two so-called "kringle" regions, and at the carboxy terminus a serine protease-type domain. We have found that the N-terminus contains several sub-domains which play functional roles, inter alia, in fibrin binding and in the in vivo clearance of the protein. Recently the recovery of another form of t-PA which lacks the native N-terminus and first kringle region has been reported, see European Published Patent Application No. 0 196 920 (published 08 October 1986). According to that report the truncated form of t-PA, which begins with Ala-160 of native human t-PA, is fibrinolytically active.

As described in greater detail hereinafter, this invention provides novel protein analogs of human t-PA which retain both kringle regions of native human t-PA, but contain modifications within the N-terminus. While in certain embodiments the modifications involve deletions in the N-terminus, the first kringle region is left intact, and the N-terminal deletion is never greater than 94 amino acids. Most embodiments involve significantly smaller deletion(s) and/or amino acid substitution(s). By retaining more of the structure of native human t-PA, it is contemplated that the proteins of this invention selectively retain more of the desirable biological activities of native human t-PA and may be less immunogenic than more drastically modified analogs of t-PA. It is therefore contemplated that the proteins of this invention possess improved fibrinolytic and pharmacokinetic profiles relative to both native human t-PA and the truncated Ala-160 t-PA, as well as other modified forms of t-PA.

The polypeptide backbone of natural human t-PA also includes four consensus Asn-linked glycosylation sites. It has been shown that two of these sites are typically glycosylated in t-PA from melanoma-derived mammalian cells, i.e. at Asn₁₁₇ and Asn₄₄₈. Asn₁₈₄ is glycosylated sometimes and Asn₂₁₈ is typically not glycosylated. t-PA from melanoma-derived mammalian cells, e.g. Bowes cells, is also referred to herein as "native" or "natural" human t-PA.

This invention, as mentioned above, involves the novel protein analogs of human t-PA that are characterized in the claims. The features of the proteins of this invention are described in greater detail below. Notwithstanding the various modifications, the numbering of amino acids as shown in the one-letter code sequence of Table 1 is retained.

### A. Modifications at the N-terminus

In one embodiment of this invention the proteins are characterized by deletion of Cys-51 through Asp-87 relative to native human t-PA. In another specific embodiment Cys-6 through Ile-86 are deleted. In a further embodiment Cys-6 through Cys-51 are deleted. In other embodiments, more conservative modifications are present in the N-terminal region of the proteins. For instance, certain proteins of this invention contain one or more amino acid deletions or substitutions within the subregions referred to in the claims. The subregions of human t-PA are the following:

| region | from | to | region | from | to |
|---|---|---|---|---|---|
| 1 | Gly-(-3) | Gln-3 | 7 | Cys-34 | Cys-43 |
| 2 | Val-4 | Lys-10 | 8 | His-44 | Ser-50 |
| 3 | Thr-11 | His-18 | 9 | Cys-51 | Cys-62 |
| 4 | Gln-19 | Leu-22 | 10 | Gln-63 | Val-72 |
| 5 | Arg-23 | Arg-27 | 11 | Cys-73 | Cys-84 |
| 6 | Ser-28 | Tyr-33 | 12 | Glu-85 | Thr-91 |

The claimed modifications within the N-terminus are described in greater detail hereinafter.

### B. Modifications at N-linked Glycosylation Sites

The protein variants of this invention further contain no N-linked carbohydrate moieties or are only partially glycosylated relative to natural human t-PA. A "partially glycosylated" protein, as the phrase is used herein, means a protein which contains fewer N-linked carbohydrate moieties than does fully-glycosylated native human t-PA. This absence of glycosylation or only partial glycosylation results from amino acid substitution or deletion at one or more of the consensus N-linked glycosylation recognition sites present in the native t-PA molecule. We have found that variant proteins of this invention embodying such modification at one or more N-linked glycosylation sites retain t-PA-type thrombolytic activity with greater fibrinolytic activity in certain cases, may be more readily produced in more homogeneous form than native t-PA, and in many cases have longer in vivo half-lives than native t-PA.

N-linked glycosylation recognition sites are presently believed to comprise tripeptide sequences which are specifically recognized by the appropriate cellular glycosylation enzymes. These tripeptide sequences are either asparagine-X-threonine or asparagine-X-serine, where X is usually any amino acid. Their location within the t-PA peptide sequence is shown in Table 1. A variety of amino acid substitutions or deletions at one or more of the three positions of a glycosylation recognition site results in non-glycosylation at the modified sequence. By way of example, Asn₁₁₇ and Asn₁₈₄ of t-PA have both been replaced with Thr in one embodiment and with Gln in another embodiment. At least in the case of the double Gln replacement, the resultant glycoprotein (Gln₁₁₇Gln₁₈₄) should contain only one N-linked carbohydrate moiety (at Asn₄₄₈) rather than two or three such moieties as in the case of native t-PA. Those skilled in the art will appreciate that analogous glycoproteins having the same Asn₄₄₈ monoglycosylation may be prepared by deletion of amino acids or substitution of other amino acids at positions 117 and 184 and/or by deleting or substituting one or more amino acids at other positions within the respective glycosylation recognitions sites, e.g. at Ser₁₁₉ and Ser₁₈₆, as mentioned above and/or by substitution, or more preferably by deletion, at one or more of the "X" positions of the tripeptide sites. In another embodiment Asn at positions 117, 184 and 448 are replaced with Gln. The resultant variants should contain no N-linked carbohydrate moieties, rather than two or three such moieties as in the case of native t-PA. In other embodiments, potential glycosylation sites have been modified individually, for instance by replacing Asn, e.g. with Gln, at position 117 in one presently preferred embodiment, at position 184 in another embodiment and at position 448 in still another embodiment. This invention encompasses such non-glycosylated, monoglycoslyated, diglycosylated and triglycosylated t-PA variants.

Exemplary modifications at one or more of the three consensus N-linked glycosylation sequences, R¹, R² and R³, as found in various embodiments of this invention are depicted below:

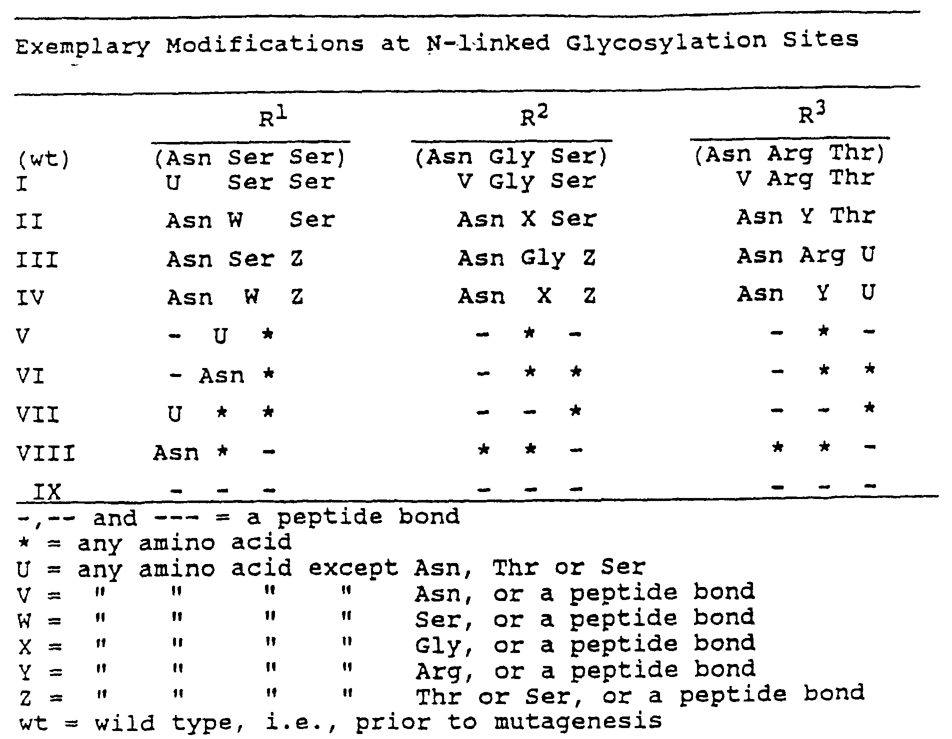

### C. Modification at the Arg-275/Ile-276 Cleavage Site

In one aspect of this invention the variants are optionally modified at the proteolytic cleavage site spanning Arg-275 and Ile-276 by virtue of deletion of Arg-275 or substitution of another amino acid, preferably an amino acid other than Lys or His, for the Arg. Thr is at present an especially prefered replacement amino acid for Arg-275 in the various embodiments of this invention. Proteolytic cleavage at Arg-275 of native t-PA yields the so-called "two-chain" molecule, as is known in the art. Proteins of this invention which are characterized by modification at this cleavage site may be more readily produced in more homogeneous form than the corresponding protein without the cleavage site modification, and perhaps more importantly may possess an improved fibrinolytic profile and pharmacokinetic characteristic.

This invention thus provides a family of novel thrombolytic proteins related to human t-PA. This family comprises several genera of proteins.

In one embodiment the proteins are characterized by a peptide sequence as characterized in the claims, wherein Arg-275 is deleted or is replaced by a different amino acid, preferably other than lysine or histidine, and at least one of the consensus Asn-linked glycosylation sites is deleted or is modified to other than a consensus Asn-linked glycosylation sequence. Exemplary proteins of this embodiment are depicted in Table 1 below. The proteins of this invention are analogs of t-PA characterized by the various modifications or combinations of modifications as disclosed herein, which may also contain other variations, e.g. allelic variations or additional deletion(s), substitution(s) or insertion(s) of amino acids which still retain thrombolytic activity, so long as the DNA encoding those proteins (prior to the modification of the invention) is still capable of hybridizing to a DNA sequence encoding human t-PA under stringent conditions.

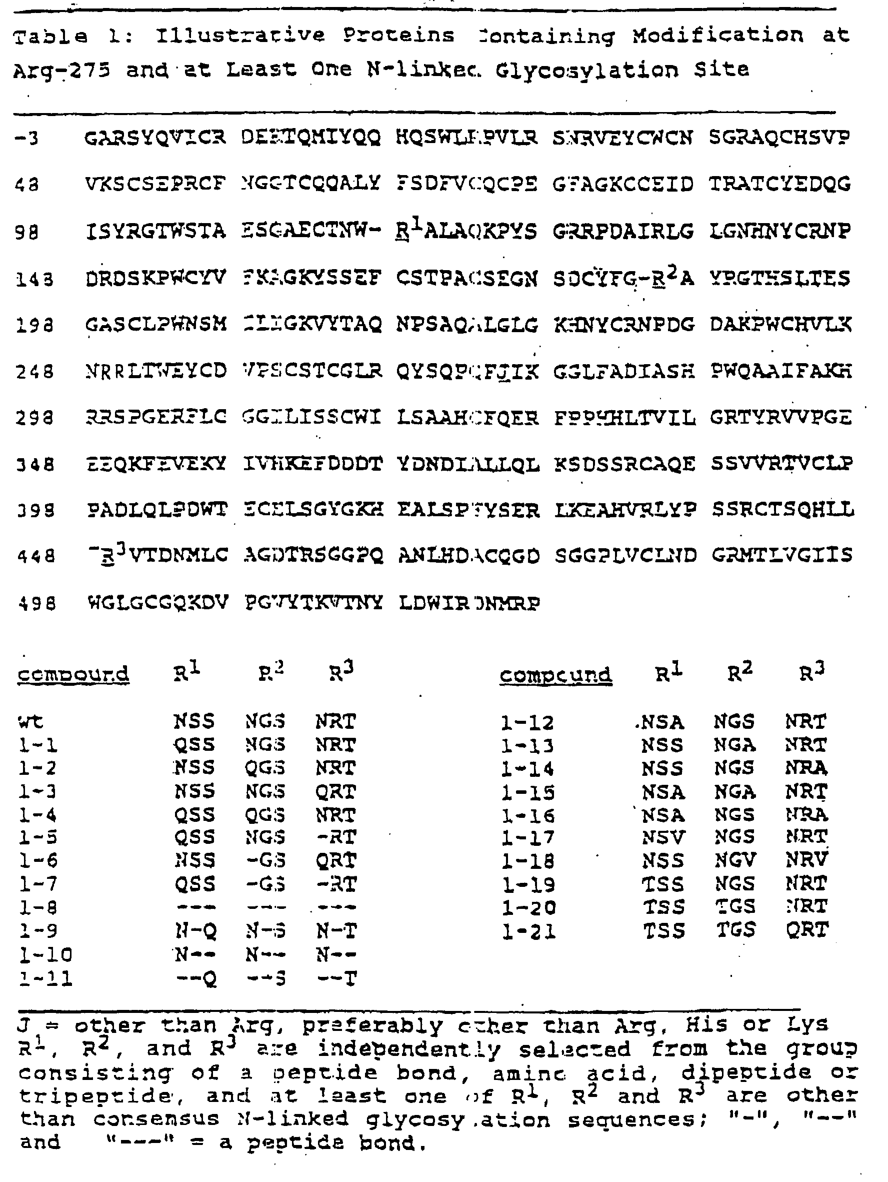

In a second embodiment the proteins are characterized by a peplide sequence substantially the same as the peptide sequence of human t-PA wherein one or more amino acids are deleted within the N-terminal region from Val-4 to Val-72 and wherein (a) one or more Asn-linked glycosylation sites are deleted or otherwise modified to other than a consensus Asn-linked glycosylation site, and (b) Arg-275 is optionally deleted or replaced by a different amino acid, preferably other than lysine or histidine. Exemplary proteins of this embodiment are shown below

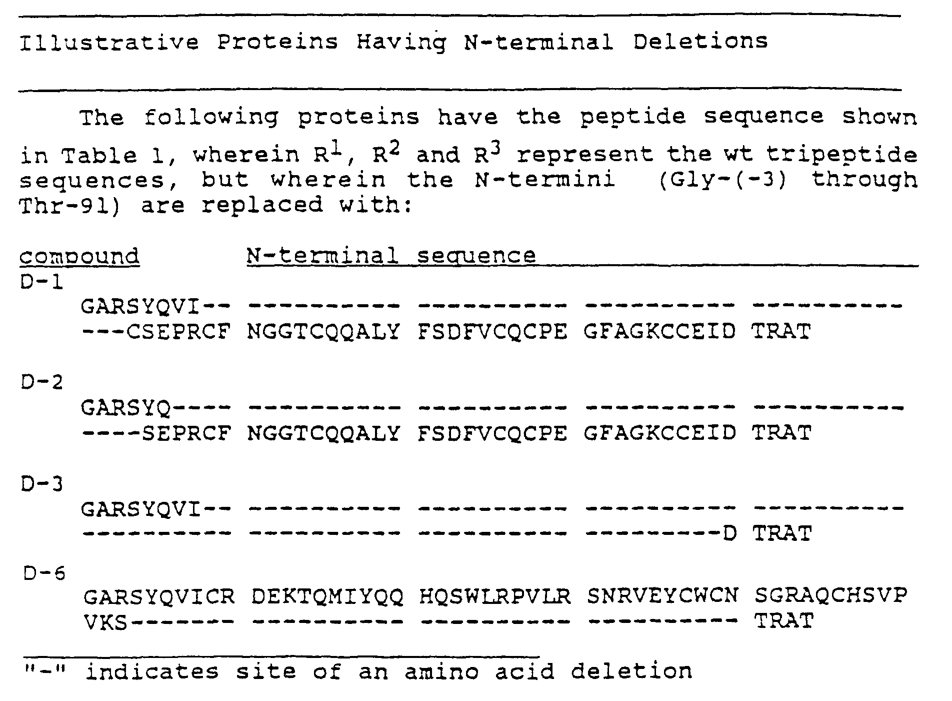

This embodiment includes a subgenus of proteins wherein 1 to about 69 amino acids are deleted from the region Val-4 through Val-72 and one or more of the Asn-linked glycosylation sites are deleted or otherwise modified to other than a consensus Asn-linked glycosylation sequence as previously described. Also included is a subgenus of compounds wherein 1 to about 69 amino acids are deleted from the region Val-4 through Val 72, and Arg-275 is deleted or replaced with a different amino acid, preferably other than lysine or histidine. A further subgenus of this embodiment is characterized by a deletion of 1 to about 69 amino acids from within the region Val-4 through Val-72 deletion, or modification of one or more of the Asn-linked glycosylation sites (see e.g. the table on page 6) and deletion of Arg-275 or replacement thereof with a different amino acid. Exemplary proteins of these subgenera are depicted in Tables 2 and 2.5, below.

This embodiment also includes a subgenus of proteins wherein the N-terminal deletion comprises a deletion of one or more amino acids from within the region Val-4 through Ser-50. Also included is a subgenus of proteins wherein one or more amino acids are deleted from within the region Val-4 through Ser-50 and one or more glycosylation sites are modified as previously described. A further subgenus comprises proteins having a deletion of amino acids from within the region Val-4 through Ser-50, wherein Arg-275 is deleted or replaced with another amino acid. Additionally included is a subgenus having deletion of one or more amino acids from within the region Val-4 through Ser-50 and wherein both of (a) one or more glycosylation sites, and (b) Arg-275, are optionally modified as previously described. Exemplary proteins of these subgenera are depicted in Table 3, below, as well as in Tables 2 and 2.5.

**Table 2:**

| Illustrative Proteins Containing a Deletion of one or more Amino Acids at the N-Terminus and Modification at Least One N-Linked Glycosylation Site and, optionally, at Arg-275. | | | | | |
|---|---|---|---|---|---|
| (for general sequence, see Table 1) | | | | | |

| compound | J | R¹ | R² | R³ | amino terminus |
|---|---|---|---|---|---|
| (wt) | R | NSS | NGS | NRT | Val-4 thru Val-72 |
| 2-0 | R | NSS | NGS | NRT | * |
| 2-1 | R | QSS | NGS | NRT | * |
| 2-2 | R | NSS | QGS | NRT | * |
| 2-3 | R | NSS | NGS | QRT | * |
| 2-4 | R | NSS | QGS | QRT | * |
| 2-5 | R | QSS | NGS | QRT | * |
| 2-6 | R | QSS | QGS | NRT | * |
| 2-7 | R | QSS | QGS | QRT | * |
| 2-8 | R | QSS | NGS | -RT | * |
| 2-9 | R | NSS | -GS | QRT | * |
| 2-10 | R | QSS | -GS | -RT | * |
| 2-11 | R | - | - | - | * |
| | | | | | |
| 2-12 | - | wt | wt | wt | * |
| 2-13 | G | wt | wt | wt | * |
| 2-14 | A | wt | wt | wt | * |
| 2-15 | T | wt | wt | wt | * |
| | | | | | |
| 2-16 | # | QSS | NGS | NRT | * |
| 2-17 | # | NSS | QGS | NRT | * |
| 2-18 | # | NSS | NGS | QRT | * |
| 2-19 | # | NSS | QGS | QRT | * |
| 2-20 | # | QSS | NGS | QRT | * |
| 2-21 | # | QSS | QGS | NRT | * |
| 2-22 | # | QSS | QGS | QRT | * |
| 2-23 | # | QSS | NGS | -RT | * |
| 2-24 | # | NSS | -GS | QRT | * |
| 2-25 | # | QSS | -GS | -RT | * |
| 2-26 | # | - | - | - | * |
| | | | | | |
| 2-27 | R | U Ser Ser | wt | wt | * |
| 2-28 | # | U Ser Ser | wt | wt | * |
| 2-29 | R | Asn W Ser | wt | wt | * |
| 2-30 | # | Asn W Ser | wt | wt | * |
| 2-31 | R | Asn Ser Z | wt | wt | * |
| 2-32 | # | Asn Ser Z | wt | wt | * |
| 2-33 | R | Asn W Z | wt | wt | * |
| 2-34 | # | Asn W Z | wt | wt | * |
| 2-35 | R | - U ^ | wt | wt | * |
| 2-36 | # | - U ^ | wt | wt | * |
| 2-37 | R | - Asn ^ | wt | wt | * |
| 2-38 | # | - Asn ^ | wt | wt | * |
| 2-39 | R | U ^ ^ | wt | wt | * |
| 2-40 | # | U ^ ^ | wt | wt | * |
| 2-41 | R | Asn ^ - | wt | wt | * |
| 2-42 | # | Asn ^ - | wt | wt | * |
| 2-43 | R | - | wt | wt | * |
| 2-44 | # | - | wt | wt | * |
| * indicates a N-terminus which is different at at least one amino acid relative to the wt N-terminus, illustrative examples of which are depicted below in Table 2.5 and in Tables 3-5 & 6-B; "#" represents a peptide bond or an amino acid other than Arginine (R); "-" represents a peptide bond; U is any amino acid except Asn, Thr or Ser; W is a peptide bond or any amino acid except Ser; Z is a peptide bond or any amino acid except Thr or Ser; ^ is any amino acid; and wt indicates wild type. | | | | | |

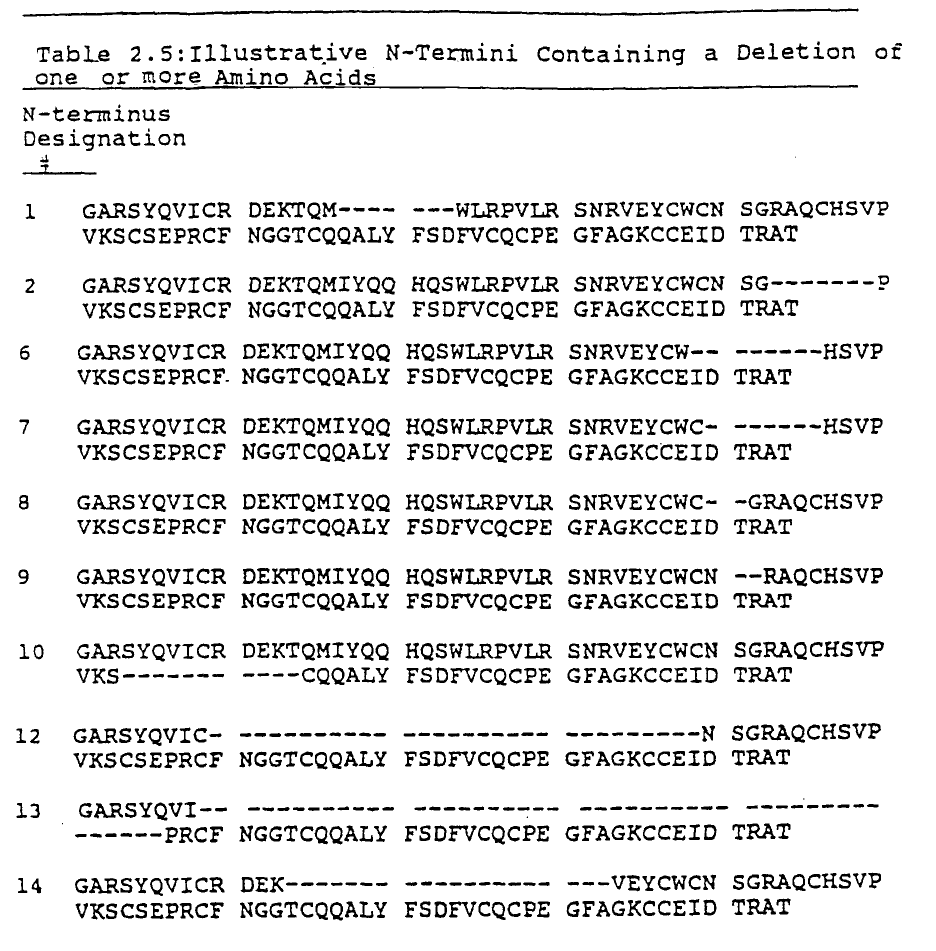

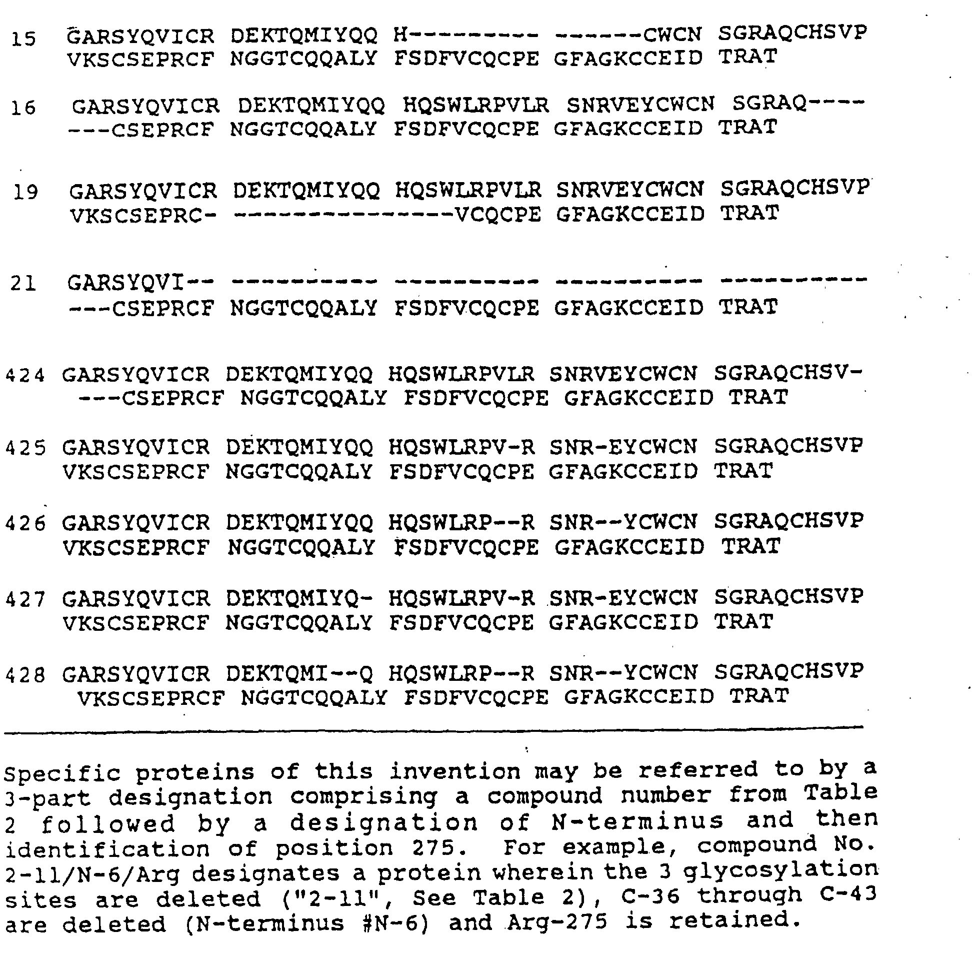

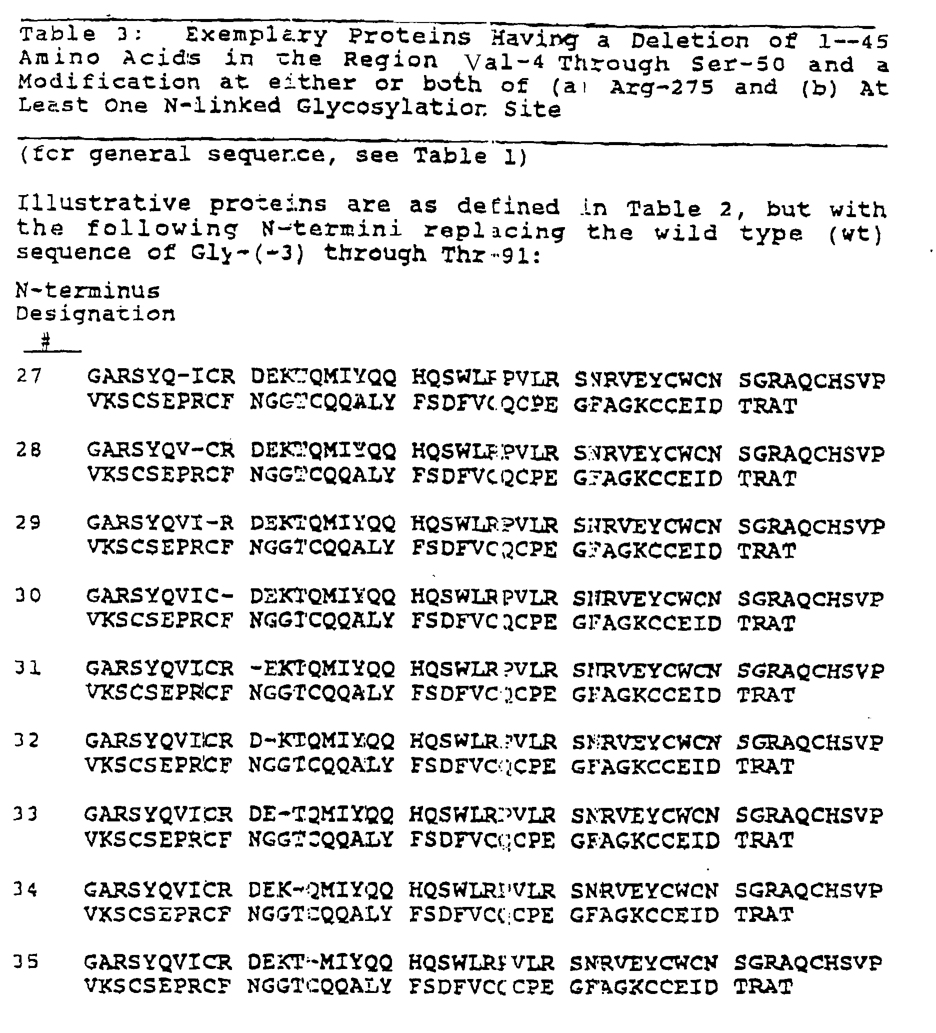

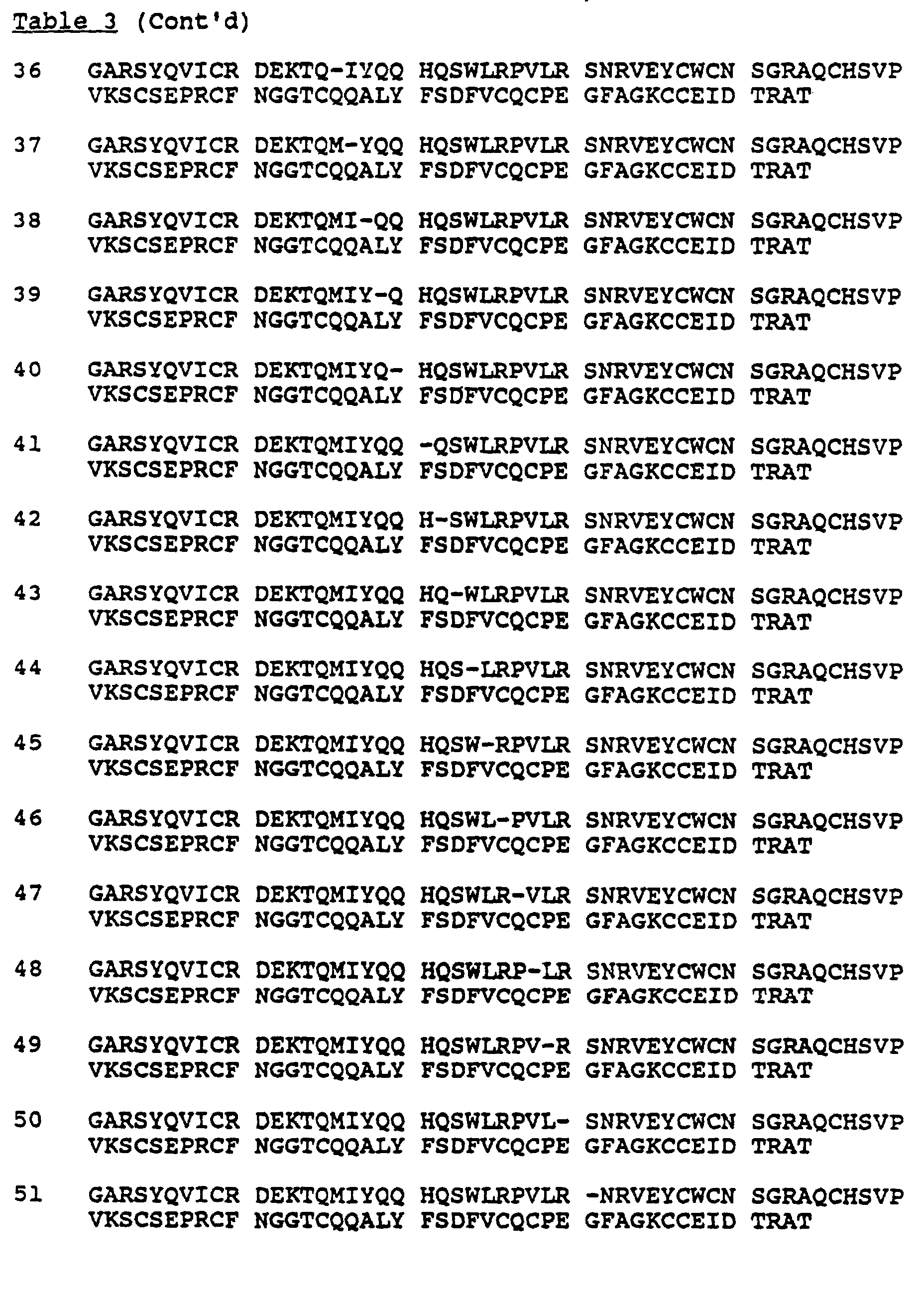

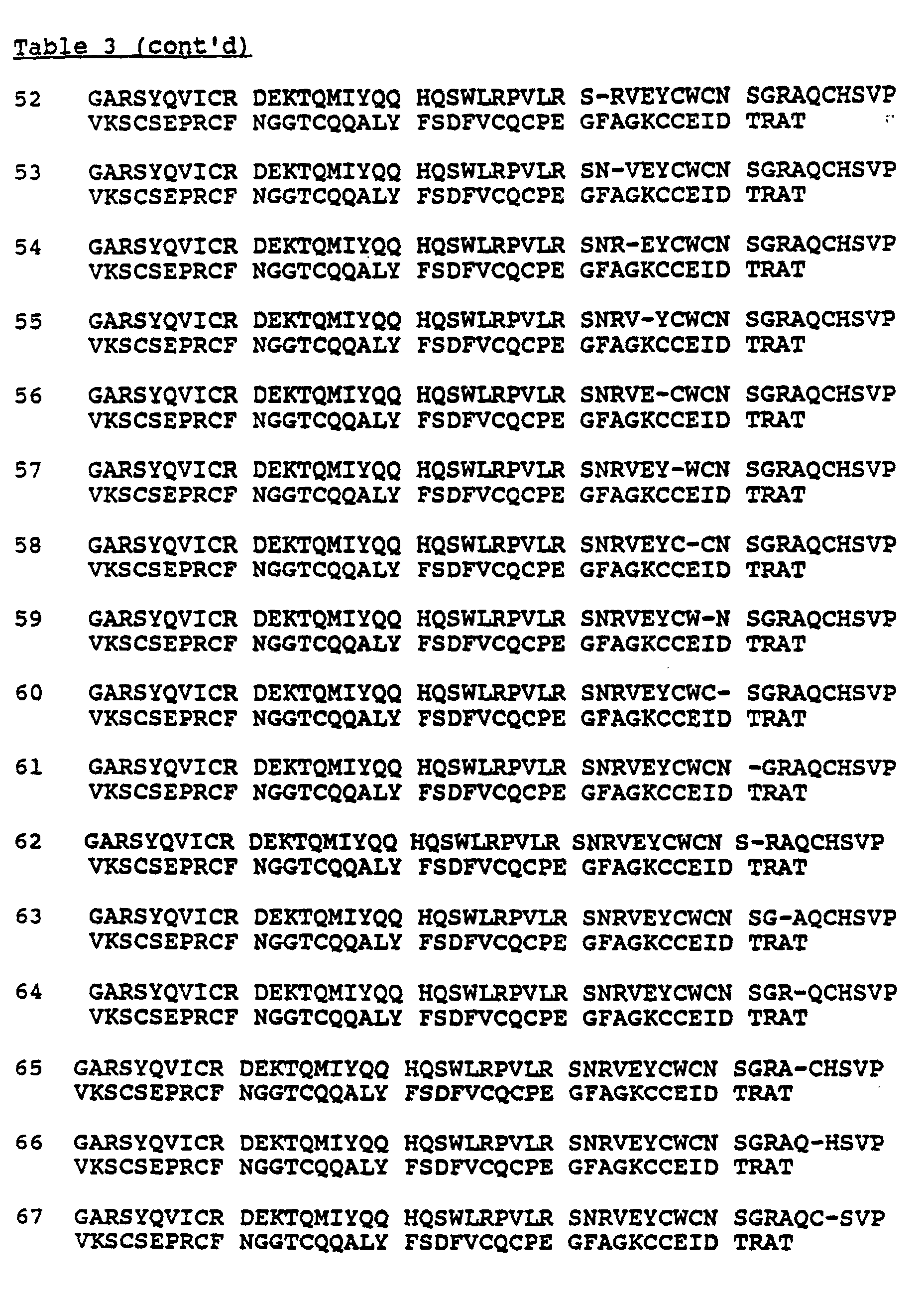

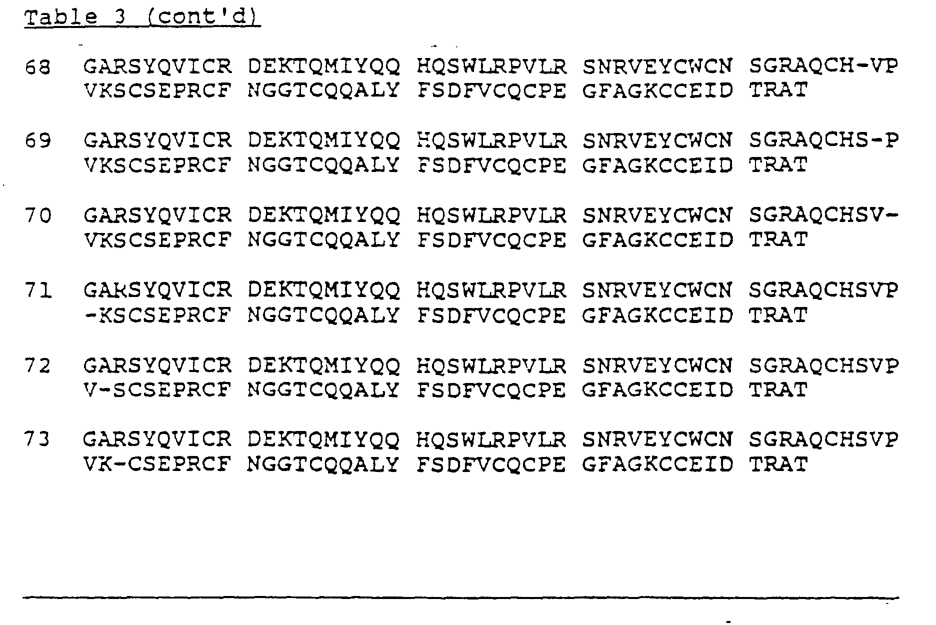

With reference to the modified N-termini depicted in Table 3, it should be understood that more than one amino acid may be deleted. Where multiple amino acids are deleted, they may be adjacent to one another, or separated by one or more other amino acids. In designating compounds with such N-termini we indicate the size of the deletion by "Δn" where "n" is the number of amino acids deleted. For example, N-terminus #27 wherein one amino acid is deleted as shown in Table 3 may be referred to as "N-27Δ1" Where two amino acids are deleted, e.g. Y-4 and I-5, the N-terminus is referred to as "N-27Δ2", etc. Where combinations of deletions are made, e.g. wherein Y-4, I-5 and D-8 are deleted, the N-terminus may be referred to as N-27Δ2, N-31Δ1. As indicated in the text following Table 2.5, specific compounds are designated by a 3-part code comprising a compound number from Table 2 followed by a designation of N-terminus #, e.g. from Table 2.5, and then identification of the status of position 275. Compound 2-26/N-N-27Δ2/N-31Δ1 thus designates the protein wherein all three glycosylation sites; R-275; S-1, Y-2 and I-5 are

This embodiment further includes a subgenus of proteins wherein one or more, amino acids are deleted from the region Val-4 through Val-72. Proteins of this subgenus may optionally be modified such that Arg-275 is deleted or replaced with a different amino acid, preferably other than lysine or histidine. Proteins of this subgenus are modified such that one or more N-linked glycosylation sites are abolished, as previously described.

Exemplary proteins of this subgenus are similar to those depicted in Tables 2 and 3, but contain N-termini such as those depicted in Table 4, below.

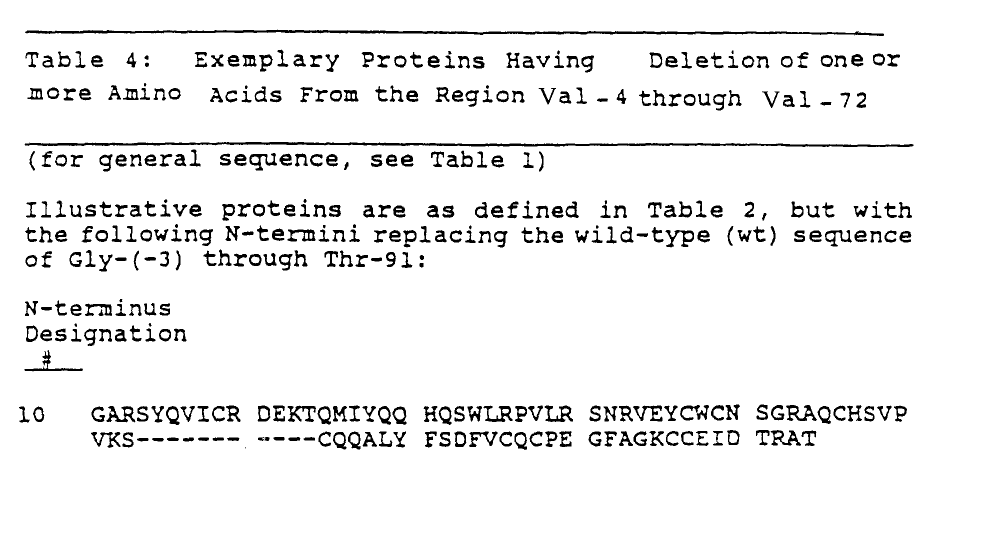

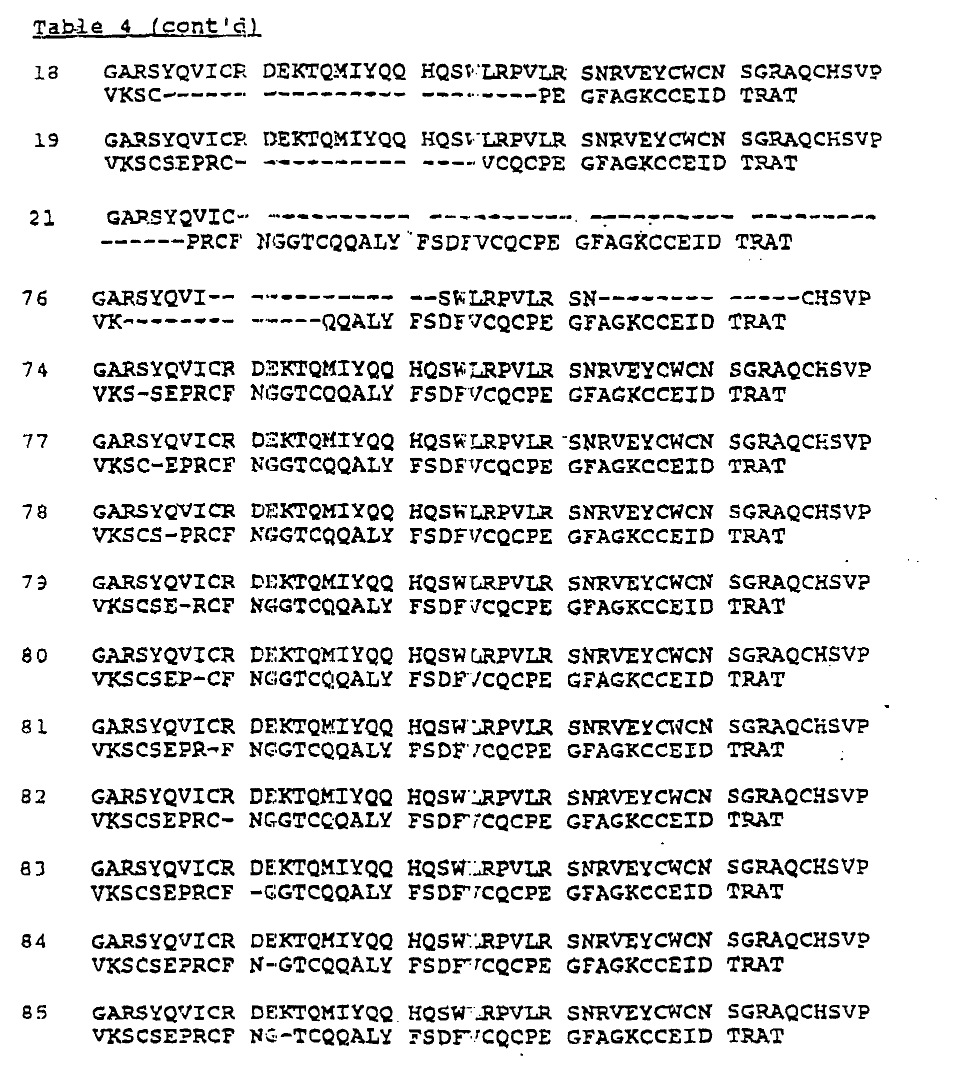

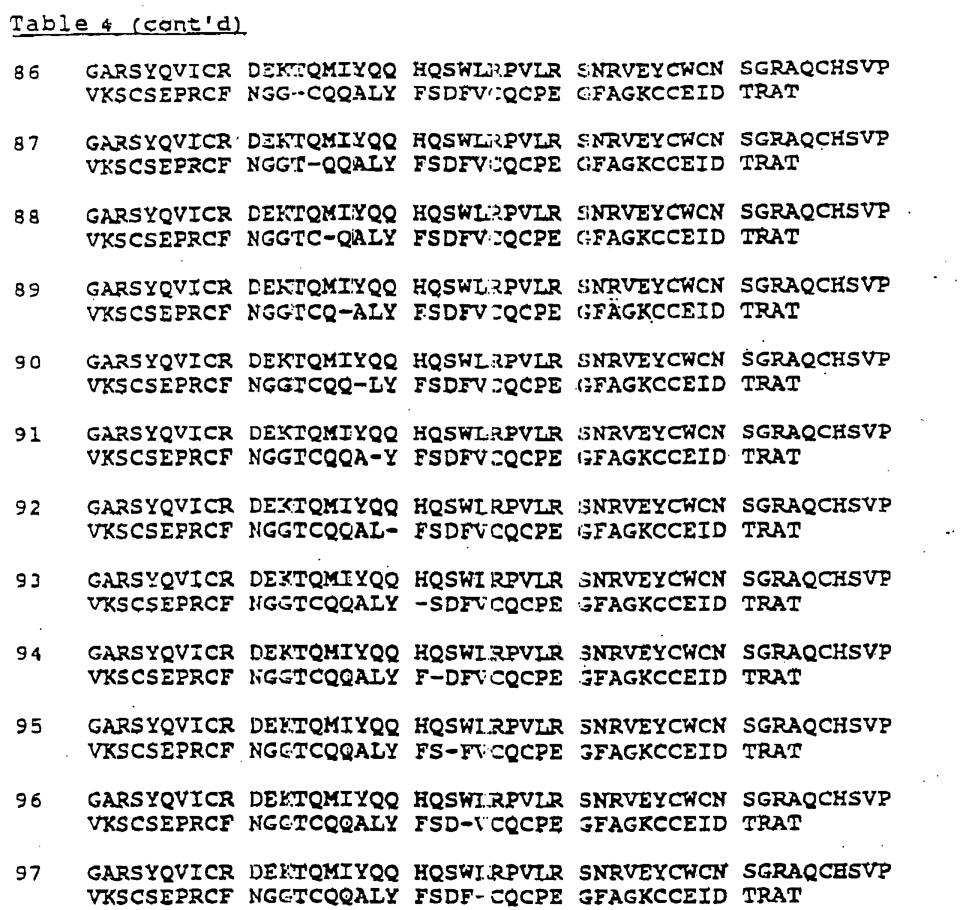

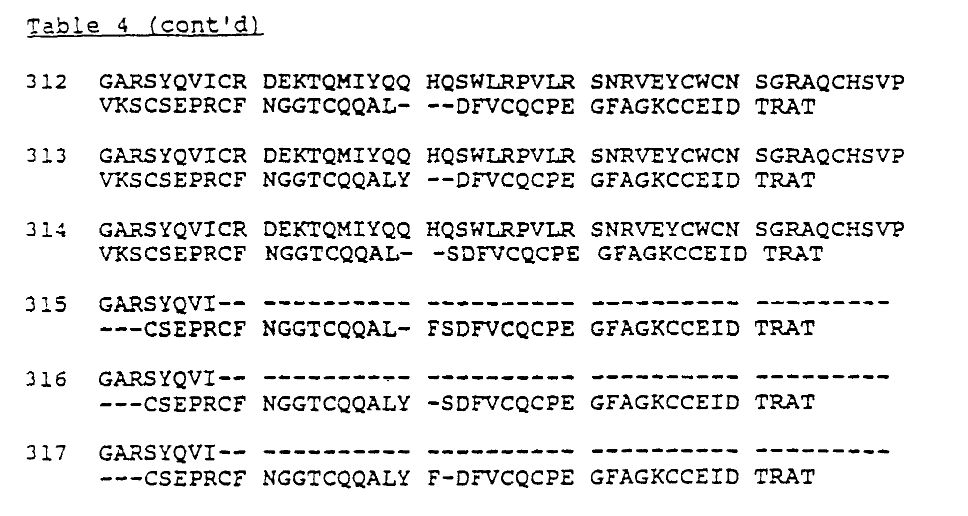

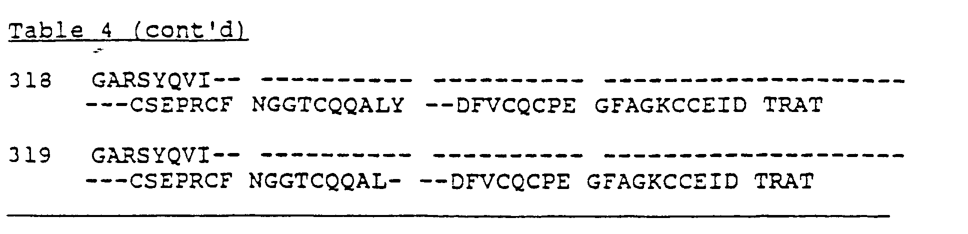

With reference to Table 4, above, it should be noted that several subclasses of proteins are disclosed. For example, proteins containing a deletion of 1-47 amino acids (individually, consecutively, or in combination) from Val-4 through Val-72 are depicted, as are proteins containing a deletion of 1-22 amino acids from Cys-51 through Val 72 and a deletion of one or more amino acids within the region Val-4 through Cys-51 (See N-76). With reference to compounds containing an N-terminus selected from N-termini N-76 through N-97 it should be understood that more than one amino acid may be deleted. For example, N-77 wherein one amino acid is deleted, as shown in Table 4, may be referred to as "N-77Δ1". Where six amino acids are deleted, e.g. S-52 through F-57, the N-terminus is referred to as "N-77Δ6", etc. Specific proteins are designated as described following Tables 2 and 3. Examples wherein the three glycosylation sites and Arg-275 are deleted are shown below:

| Compound Designation | Deletions |
|---|---|
| 2-26/N-10/- | C-51 through T-61 |
| 2-26/N-74Δ6/- | C-51 through C-56 |
| 2-27/N-74Δ1, N-76Δ6/- | C-51,E-53 through N-58 |

Illustrative compounds which are modified at the first N-linked glycosylation site, here by replacing Asn-117 with Gln, are listed below:
2-1/N-424/Arg
2-1/N-425/Arg
2-1/N-426/Arg
2-1/N-427/Arg
2-1/N-428/Arg
2-1/N-63Δ2,N-92Δ3/Arg
2-1/N-63Δ1,N-92Δ2/Arg
2-1/N-63Δ1,N-92Δ1/Arg
Illustrative compounds with the same deletions as above, but which are also modified at all three glycosylation sites, here by replacement of Asn's with Gln's, are listed below:
2-7/N-424/Arg
2-7/N-425/Arg
2-7/N-426/Arg
2-7/N-427/Arg
2-7/N-428/Arg
2-7/N-63Δ2,N-92Δ3/Arg
2-7/N-63Δ1,N-92Δ2/Arg
2-7/N-63Δ1,N-92Δ1/Arg
Thus, this embodiment further includes a subgenus c proteins wherein one or more deletions of less than about 20 amino acids are present within the region Val-4 through Val-72 Proteins of this subgenus are modified at one or more of the Asn-linked glycosylation sites and, optionally, at Arg-275. Exemplary proteins of this subgenus are similar to those depicted in Tables 2 through 4, but contain in place of the wild type N-terminus. an N-terminus such as those depicted in Table 5, below. Additional exemplary compounds of this subgenus are also listed above by their 3-part code designations.

In a third embodiment the proteins are characterized by a peptide sequence substantially the same as the peptide sequence of human t-PA wherein different amino acids are substituted for amino acids in the region Arg-23 to Val-72. This embodiment includes a subgenus of compounds characterized by replacement of one or more amino acids within the above mentioned N-terminus and by modification at Arg-275 as previously described. This embodiment of compounds is also characterized by the modification, as previously described, at one or more of the consensus Asn-linked glycosylation sites. A further subgenus of this embodiments is characterized by substitution of one or more amino acids within the N-terminus, and modifications as previously described, at both Arg-275 and at one or more of the N-linked glycosylation sites. In a further aspect, one to about eleven, preferably one to about 6 amino acids are replaced within one or more of the following regions.

| region | from | to | region | from | to |
|---|---|---|---|---|---|
| 5 | Arg-23 | Arg-27 | 7 | Cys-34 | Cys-43 |
| 6 | Ser-23 | Tyr-33 | 8 | His-44 | Ser-50 |
| | | | 9 | Cys-51 | Cys-62 |
| | | | 10 | Gln-63 | Val-72 |

In a further aspect of this embodiment, the substitution(s) is/are present in one or more of the following regions: N-3' through C-42, and H-44 through S-50. In an additional aspect of this embodiment, the N-terminus is modified, again, by substitution for one to about eleven, preferably one to about six amino acids in one or more of the above defined regions, and is further modified by deletion of one to 50, preferably 1 to about 45, and more preferably 1 to about 15, amino acids.

Illustrative amino acid substitutions are shown in Table 6-A below, and exemplary proteins are depicted in Table 6-B. Of the replacement amino acids for R-40, A-41 and Q-42, presented in Table 6-A, S is a preferred replacement for R-40, and V and L are preferred replacements for A-41 and Q-42, respectively. It should be noted that proteins of this invention embodying the substitutions identified for R-40, A-41 and Q-42 in Table 6A are in combination with modifications at least one glycosylation site and, optionally, with other substitution(s) and/or deletions within the N-terminus and/or with modifications at R275. It is contemplated that to the extent that our proteins are modified by substitution rather than deletion, our proteins retain more of the native t-PA conformation and selectively retain more of the desirable biological activities of native t-PA.

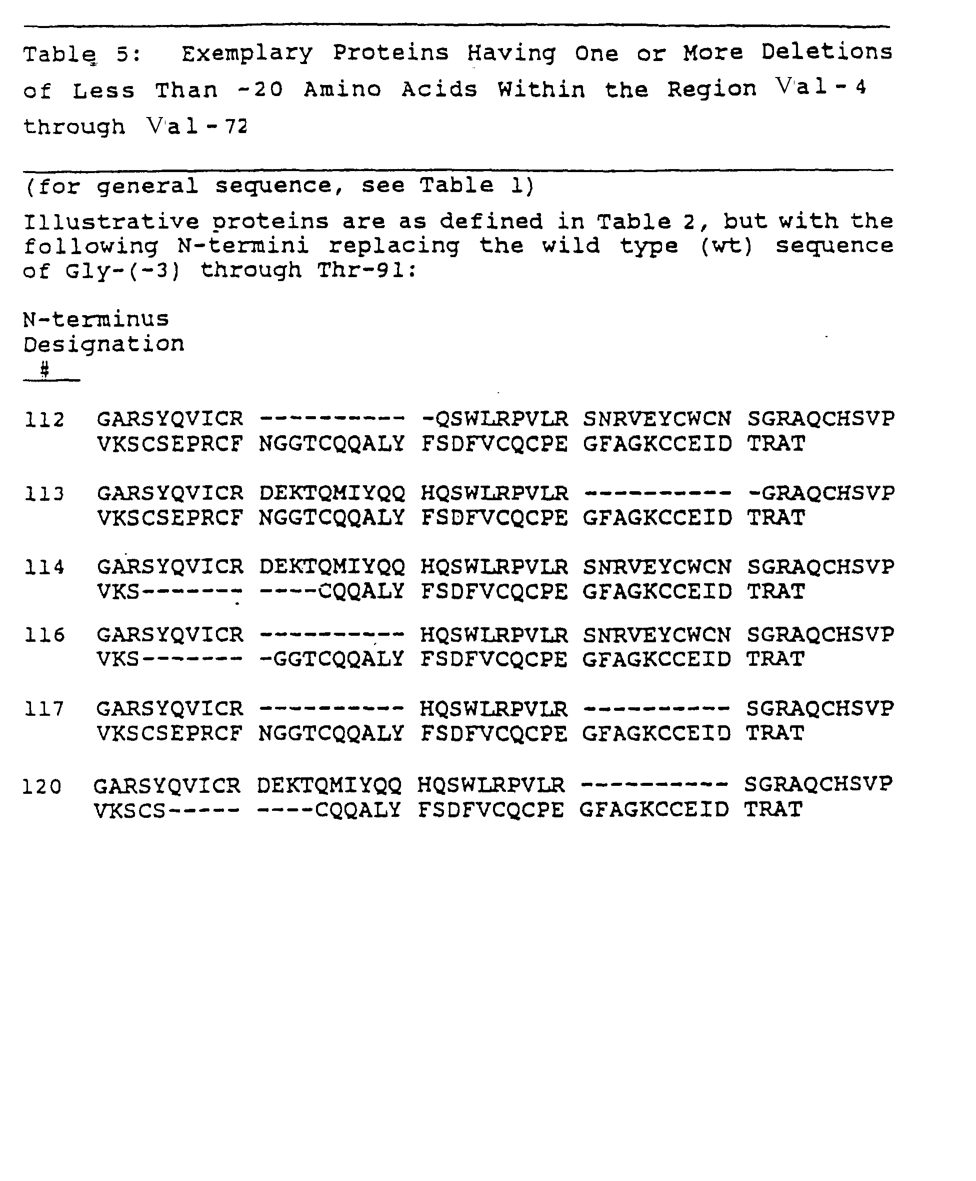

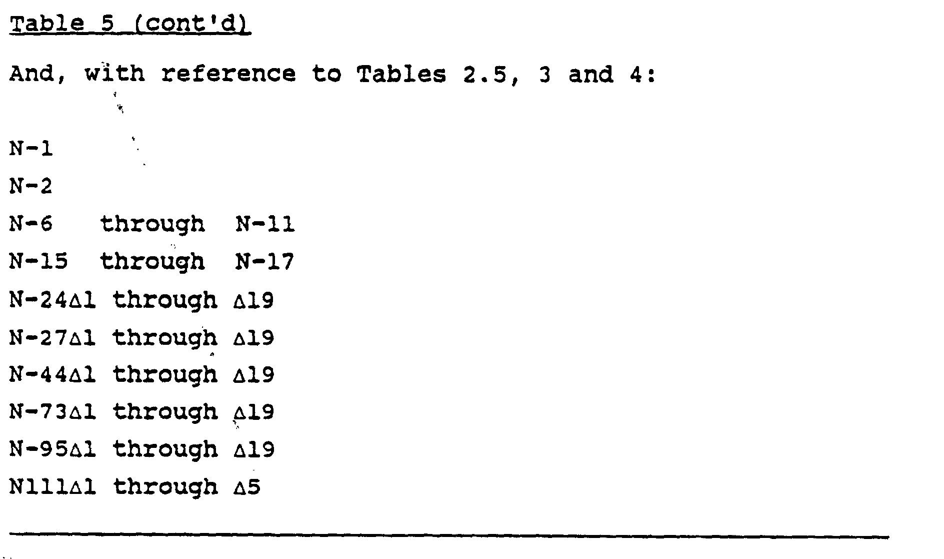

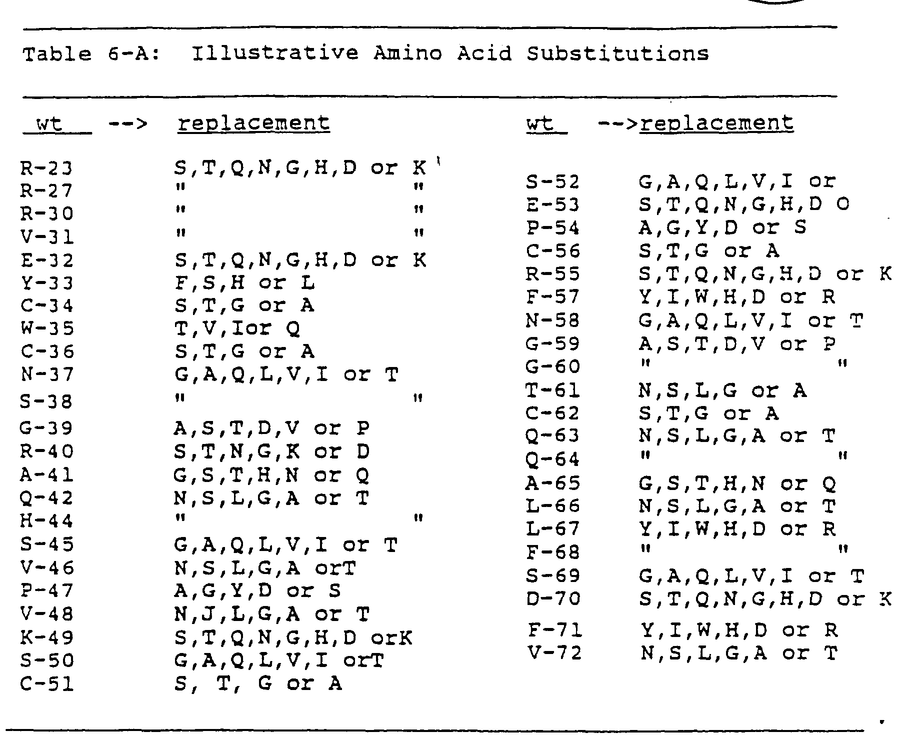

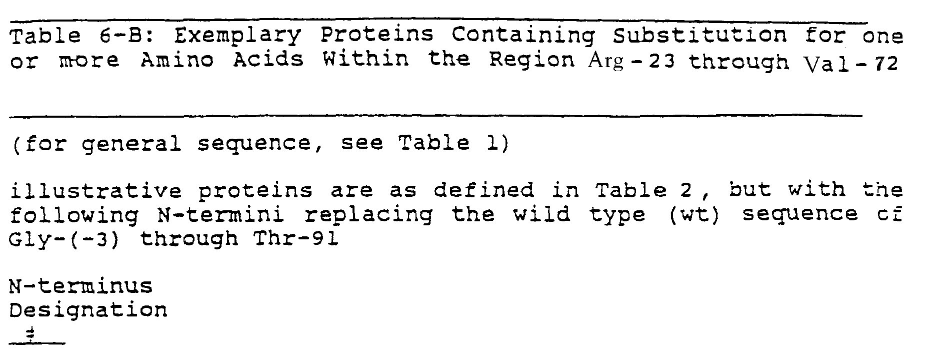

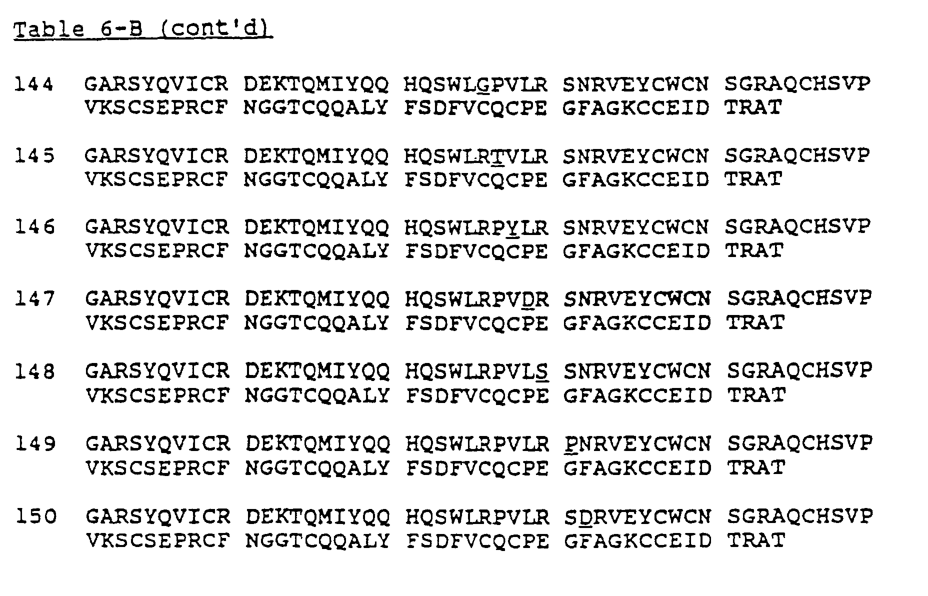

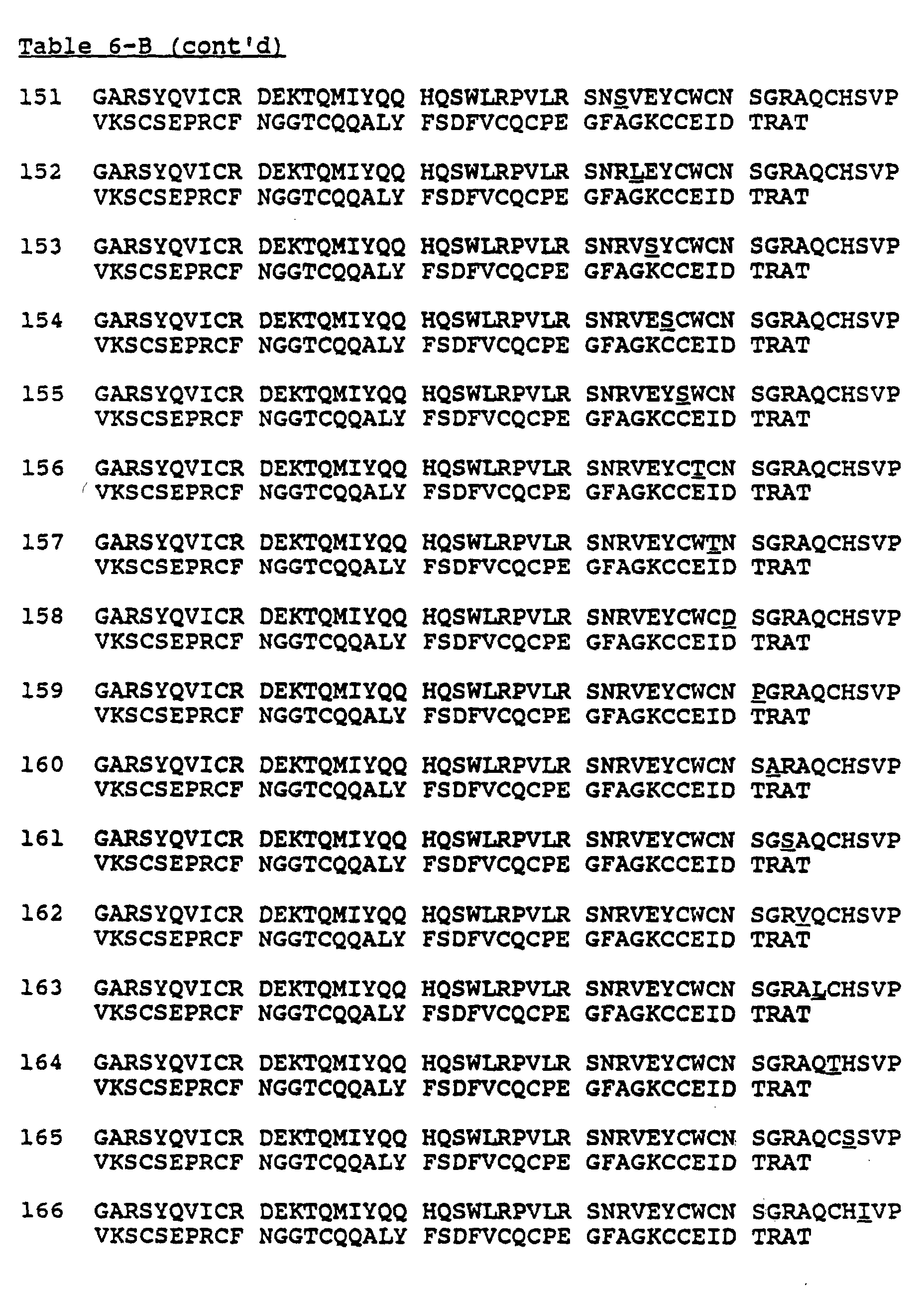

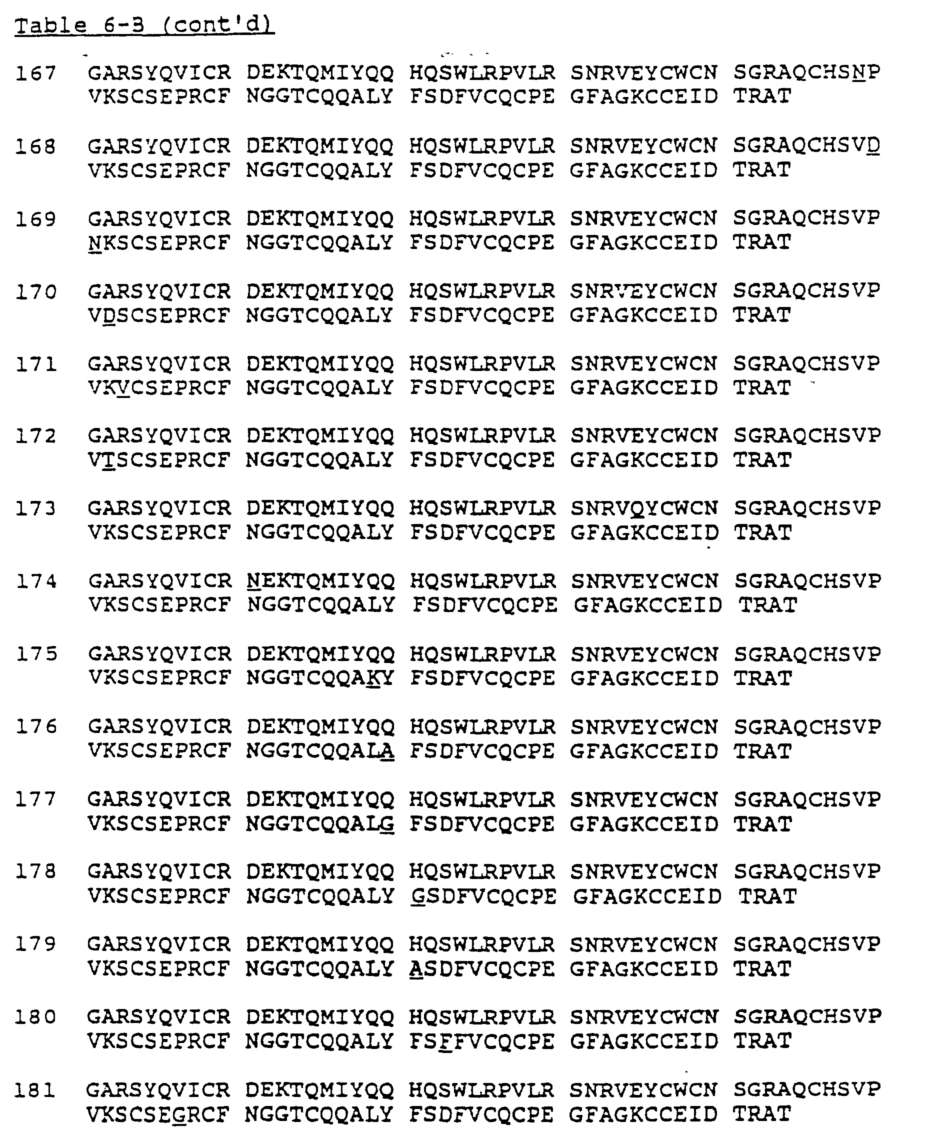

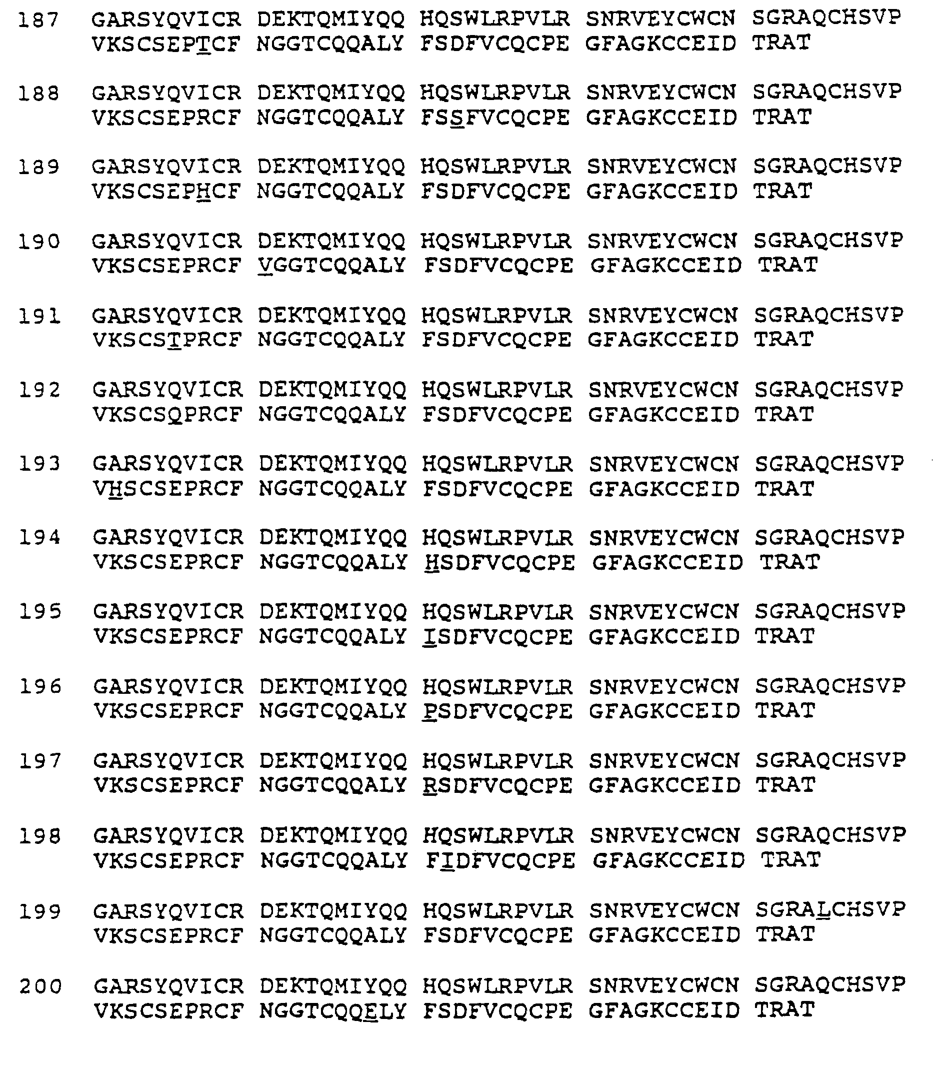

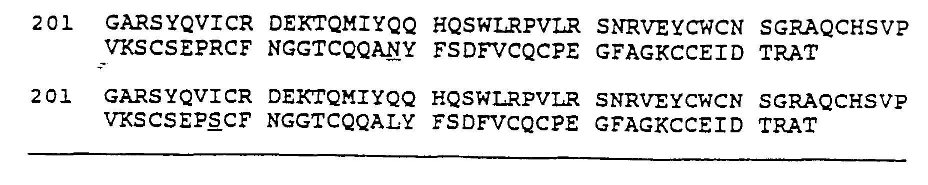

Proteins of this invention embodying amino acid substitution(s) may be designated by a 3-part code as previously described. It should be understood of course that more than one wt amino acid may be replaced. In designating compounds with such N-termini we indicate the number of substitutions by "sn" where "n" is the number of amino acids replaced, e.g. with the replacement amino acids such as (but not limited to) those depicted in Table 6-A. For example, N-terminus #N14451 designates N-terminus 144 as depicted in Table 6-B, while N-terminus #N14454 designates that N-terminus wherein R-23 is replaced with G and the following three wt amino acids are replaced with other amino acids. Proteins of this embodiment containing multiple amino acid substitutions may be designated by a string of N-terminus designations indicating specific replacements, as follows:
Illustrative compounds with multiple N-terminal substitutions, with Asn-117 replaced with Gln and Arg-275 replaced with Thr:

| compound # | substitutions: wt replacement | |
|---|---|---|
| 2-16/N-161,N-165/Thr | R-40 | S |
| | H-44 | S |
| 2-16/N-159,N-161/Thr | S-38 | P |
| | R-40 | S |
| 2-16/N-161,N-163/Thr | R-40 | S |
| | Q-42 | L |
| 2-16/N-161,N-172/Thr | R-40 | S |
| | K-49 | T |
| 2-16/N-165,N-170/Thr | H-44 | S |
| | K-49 | D |
| 2-16/N-173,N-165/Thr | E-32 | Q |
| | H-44 | S |
| 2-16/N-165,N-172/Thr | H-44 | S |
| | K-49 | T |
| 2-16/N-148,N-151/Thr | R-27 | S |
| | R-30 | S |
| 2-16/N-161,N-170/Thr | R-40 | S |
| | K-49 | D |
| 2-16/N-153,N-161/Thr | E-32 | S |
| | R-40 | S |
| 2-16/N-175,N-176/Thr | L-66 | K |
| | Y-67 | A |
| 2-16/N-176,N-178/Thr | Y-67 | A |
| | F-68 | G |
| 2-16/N-188,N-201/Thr | L-66 | N |
| | D-70 | S |
| 2-16/N-189,N-190/Thr | R-55 | H |
| | N-58 | V |
| 2-16/N-191,N-189/Thr | E-53 | T |
| | R-55 | H |
| 2-16/N-193,N-192/Thr | K-49 | H |
| | E-53 | Q |
| 2-16/N-161,N-175/Thr | R-40 | S |
| | L-66 | K |
| 2-16/N-148,N-194/Thr | R-27 | S |
| | F-68 | H |
| 2-16/N-144,N-195/Thr | R-23 | G |
| | F-68 | I |
| 2-16/N-151,N-196/Thr | R-30 | S |
| | F-68 | P |
| 2-16/N-161,N-198/Thr | R-40 | S |
| | S-69 | I |
| 2-16/N-199,N-200/Thr | Q-42 | L |
| | A-65 | E |
| 2-16/N-161,N-197/Thr | R-40 | S |
| | F-68 | R |

One subgenus of particular interest is characterized by replacement of one or more of Y-67 through S-69, with optional deletion of, and/or substitution for, one or more amino acids from Arg-23 through L-66, with modification as described above at one or more glycosylation sites and, optionally, at Arg-275.

In one aspect of the invention the proteins contain at least one so-called "complex carbohydrate" sugar moiety characteristic of mammalian glycoproteins. As exemplified in greater detail below, such "complex carbohydrate" glycoproteins may be produced by expression of a DNA molecule encoding the desired polypeptide sequence in mammalian host cells. Suitable mammalian host cells and methods for transformation, culture, amplification, screening, and product production and purification are known in the art. See e.g. Gething and Sambrook, Nature 293:620-625 (1981), or alternatively, Kaufman et al., Molecular and Cellular Biology 5 (7):1750-1759 (1985) or Howley et al., U.S. Patent No. 4,419,446.

A further aspect of this invention involves t-PA variants as defined above in which each carbohydrate moiety is a processed form of the initial dolicol-linked oligosaccharide characteristic of insect cell-produced glycoproteins, as opposed to a "complex carbohydrate" substituent characteristic of mammalian glycoproteins, including mammalian derived t-PA. Such insect cell-type glycosylation is referred to herein as "high mannose" carbohydrate for the sake of simplicity. For the purpose of this disclosure, complex and high mannose carbohydrates are as defined in Kornfeld et al., Ann. Rev. Biochem. 54: 631-64 (1985). "High mannose" variants in accordance with this invention are characterized by a variant polypeptide backbone as described above which contains at least one occupied N-linked glycosylation site. Such variants may be produced by expression of a DNA sequence encoding the variant in insect host cells. Suitable insect host cells as well as methods and materials for transformation/transfection, insect cell culture, screening and product production and purification useful in practicing this aspect of the invention are known in the art. Glycoproteins so produced also differ from natural t-PA and from t-PA produced heretofore by recombinant engineering techniques in mammalian cells in that the variants of this aspect of the invention do not contain terminal sialic acid or galactose substituents on the carbohydrate moieties or other protein modifications characteristic of mammalian derived glycoproteins.

The proteins of this invention which contain no N-linked carbohydrate moieties may also be produced by expressing a DNA molecule encoding the desired variant, e.g. compounds 1-6 through 1-11 of Table 1, in mammalian, insect, yeast or bacterial host cells, with eucaryotic host cells being presently preferred. As indicated above suitable mammalian and insect host cells, and in addition, suitable yeast and bacterial host cells, as well as methods and materials for transformation/transfection, cell culture, screening and product production and purification useful in practicing this aspect of the invention are also known in the art.

Additionally, as should be clear to those of ordinary skill in this art. this invention also contemplates other t-PA variants which are characterized, instead of by amino acid deletion within the region Val-4 through Val-72, by one or more amino acid substitutions within that region, especially in the region Val-4 through Val-72, or by a combination of deletion and substitution. cDNAs encoding these compounds may be readily prepared, e.g., by methods closely analogous to the mutagenesis procedures described herein using appropriate mutagenesis oligonucleotides. The cDNAs are mutagenized at one or more of the codons for R¹, R² and R³, and Arg-275, and may be inserted into expression vectors and expressed in host cells by the methods disclosed herein. It is contemplated that these proteins will share the adavantageous pharmacokinetic properties of the other compounds of this invention, and perhaps avoid undue antigenicity upon administration in pharmacuetical preparations analogous to those disclosed herein.

As should be evident from the preceding, all variants of this invention are prepared by recombinant techniques using DNA sequences encoding the analogs which also contain fewer or no potential glycosylation sites relative to natural human t-PA and, optionally deletion or replacement of Arg-275. Such DNA sequences may be produced by conventional site-directed mutagenesis of DNA sequences encoding t-PA.

DNA sequences encoding t-PA have been cloned and characterized. See e.g., D. Pennica et al., Nature (London) 301:214(1983) and R. Kaufman et al., Mol. Cell. Biol.5(7): 1750 (1985). One clone, ATCC 39891, which encodes a thrombolytically active t-PA analog is unique in that it contains a Met residue at position 245 rather than Val. Typically, the DNA sequence encodes a leader sequence which is processed, i.e., recognized and removed by the host cell, followed by the amino acid residues of the full length protein, beginning with Gly.Ala.Arg.Ser.Tyr.Gln.. . Depending on the media and host cell in which the DNA sequence is expresed, the protein so produced may begin with the Gly.Ala.Arg amino terminus or be further processed such that the first three amino acid residues are proteolytically removed. In the latter case, the mature protein has an amino terminus comprising:
Ser.Tyr.Gln.Leu... . t-PA variants having either amino terminus are thrombolytically active and are encompassed by this invention. Variants in accord with the present invention also include proteins having either Met₂₄₅ or Val ₂₄₅, as well as other variants, e.g. allelic variations or other amino acid substitutions or deletions, which still retain thrombolytic activity.

As mentioned above, DNA sequences encoding individual variants of this invention may be produced by conventional site-directed mutagenesis of a DNA sequence encoding human t-PA or analogs or variants thereof. Such methods of mutagenesis include the MI3 system of Zoller and Smith, Nucleic Acids Res. 10:6487-6500 (1982); Methods Enzymol. 100:
468-500 (1983); and DNA 3:479-488 (1984), using single stranded DNA and the method of Morinaga et al., Bio/technology, 636-639 (July 1984), using heteroduplexed DNA. Several exemplary oligonucleotides used in accordance with such methods to effect deletions in the N-terminus or to convert an asparagine residue to threonine or glutamine, for example, are shown in Table 7. It should be understood, of course, that DNA encoding each of the glycoproteins of this invention may be analogously produced by one skilled in the art through site-directed mutagenesis using(an) appropriately chosen oligonucleotide(s). Expression of the DNA by conventional means in a mammalian, yeast, bacterial, or insect host cell system yields the desired variant. Mammalian expression systems and the variants obtained thereby are presently preferred.

The mammalian cell expression vectors described herein may be synthesized by techniques well known to those skilled in this art. The components of the vectors such as the bacterial replicons, selection genes, enhancers, promoters, and the like may be obtained from natural sources or synthesized by known procedures. See Kaufman et al., J. Mol Biol., 159:51-521 (1982); Kaufman, Proc Natl. Acad. Sci. 82:689-693 (1985).

Established cell lines, including transformed cell lines, are suitable as hosts. Normal diploid cells. cell strains derived from in vitro culture of primary tissue, as well as primary explants (including relatively undifferentiated cells such as hematopoetic stem cells) are also suitable. Candidate cells need not be genotypically deficient in the selection gene so long as the selection gene is dominantly acting.

The host cells preferably will be established mammalian cell lines. For stable integration of the vector DNA into chromosomal DNA, and for subsequent amplification of the integrated vector DNA, both by conventional methods, CHO (Chinese hamster Ovary) cells are presently preferred. Alternatively, the vector DNA may include all or part of the bovine papilloma virus genome (Lusky et al., Cell, 36:391-401 (1984) and be carried in cell lines such as C127 mouse cells as a stable episomal element. Other usable mammalian cell lines include but are not limited to, HeLa, COS-I monkey cells, mouse L-929 cells, 3T3 lines derived from Swiss, Balb-c or NIH mice, BHK or HaK hamster cells lines and the like.

Stable transformants then are screened for expression of the product by standard immunological or enzymatic assays. The presence of the DNA encoding the variant proteins may be detected by standard procedures such as Southern blotting. Transient expression of the DNA encoding the variants during the several days after introduction of the expression vector DNA into suitable host cells such as COS-I monkey cells is measured without selection by activity or immunologic assay of the proteins in the culture medium.

In the case of bacterial expression, the DNA encoding the variant may be further modified to contain different codons for bacterial expression as is known in the art and preferably is operatively linked in-frame to a nucleotide sequence encoding a secretory leader polypeptide permitting bacterial expression, secretion and processing of the mature variant protein, also as is known in the art. The compounds expressed in mammalian, insect, yeast or bacterial host cells may then be recovered, purified, and/or characterized with respect to physicochemical, biochemical and/or clinical parameters, all by known methods.

These compounds have been found to bind to monoclonal antibodies directed to human t-PA, and may thus be recovered and/or purified by immunoaffinity chromatography using such antibodies. Furthermore, these compounds possess t-PA-type enzymatic activity, i.e., compounds of this invention effectively activate plasminogen in the presence of fibrin to evoke fibrinolysis, as measured in an indirect assay using the plasmin chromogenic substrate S-2251 as is known in the art.

This invention also encompasses compositions for thrombolytic therapy which comprise a therapeutically effective amount of a variant described above in admixture with a pharmaceutically acceptable parenteral carrier. Such composition can be used in the same manner as that described for human t-PA and should be useful in humans or lower animals such as dogs, cats and other mammals known to be subject to thrombotic cardiovascular problems. It is contemplated that the compositions will be used both for treating and desirably for preventing thrombotic conditions. The exact doseage and method of administration will be determined by the attending physician depending on the potency and pharmacokinetic profile of the particular compound as well as on various factors which modify the actions of drugs, for example, body weight, sex, diet, time of administration, drug combination, reaction sensitivities and severity of the particular case.

The following examples are given to illustrate embodiments of the invention. It will be understood that these examples are illustrative, and the invention is not to be considered as restricted thereto except as indicated in the appended claims.

In each of the examples involving insect cell expression, the nuclear polyhedrosis virus used was the L-1 variant of the Autographa Californica, and the insect cell line used was the spodoptera frugiperda IPLB-SF2I cell line (Vaughn, J.L. et al., In Vitro (1977) 13, 213-217). The cell and viral manipulations were as detailed in the literature (Pennock G.D., et al., supra; Miller, D.W., Safer, P., and Miller, L.K., Genetic Engineering, Vol. 8, pages 277-298, J.K. Setlow and A. Hollaender, eds. Plenum Press, 1986). The RF mI3 vectors, mp18 and mp 11, are commercially available from New England Biolabs. However, those of ordinary skill in the art to which this invention pertains will appreciate that other viruses, strains, host cells, promoters and vectors containing the relevant cDNA, as discussed above, may also be used in the practice of each embodiment of this invention. The DNA manipulations employed are, unless specifically set forth herein, in accordance with Maniatis et al., Molecular Cloning:
A Laboratory Manual (Cold Spring Harbor, NY 1982).

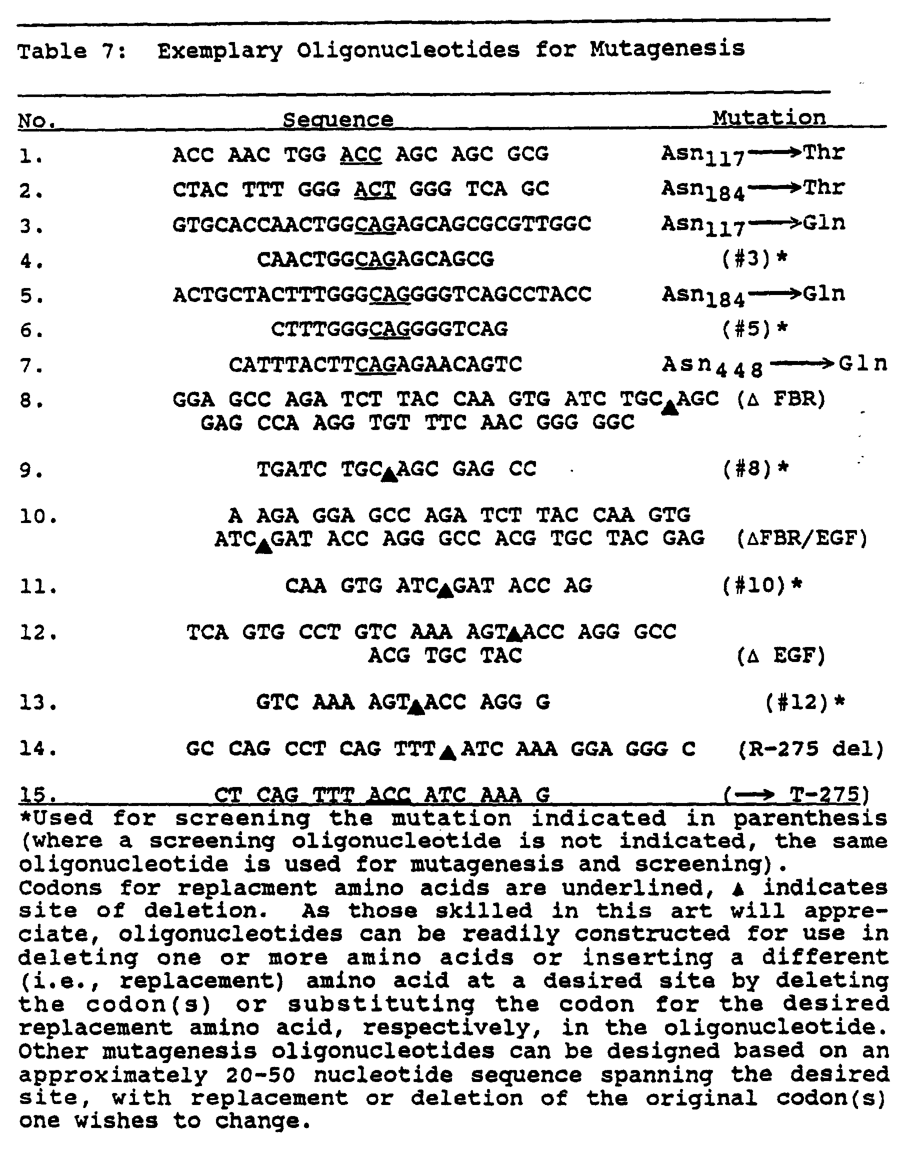

### Plasmid Derivations

Mutagenesis of cDNAs at codons for the various amino acids was conducted using an appropriate restriction fragment of the cDNA in MI3 plasmids by the method of Zoller and Smith. Deletions within the cDNA were effected by loopout mutagenesis using an appropriate restriction fragment, e.g. the SacI fragment, of the cDNA either in MI3 vectors or by heteroduplex loop-out in plasmid pSVPA4.

The plasmid pSVPA4 was constructed to allow the expression of t-PA glycoprotein in mammalian cells. This plasmid was made by first removing the DNA encoding the SV40 large T polypeptide from the plasmid pspLT5 (Zhu, Z. et al., 1984, J. Virology 51:170-180). This was accomplished by performing a total Xho I digest followed by partial Bam-HI restriction endonuclease digestion. The SV40 large T encoding region in pspLT5 was replaced with human t-PA-encoding sequence by ligating a cohesive Sall/ BamHI t-PA encoding restriction fragment, isolated by digesting plasmid J205 (ATCC No. 39568) with Sal I and BamHI, to the parent Xhol/BamHI cut vector pspLT5 prepared as described above. Consequently, t-PA will be transcribed in this vector under the control of the SV40 late promoter when introduced into mammalian cells. This final contruct is designated pSVPA4.

Plasmid pLDSG is an amplifiable vector for the expression of t-PA in mammalian cells such as CHO cells. pLDSG contains a mouse DHFR cDNA transcription unit which utilizes the adenovirus type 2 major late promoter (MLP), the simian virus 40 (SV40) enhancer and origin of replication, the SV40 late promoter (in the same orientation as the adenovirus MLP), a gene encoding tetracyclin resistance and a cDNA encoding human t-PA (Met-245) in the proper orientation with respect to the adenovirus type 2 MLP. The preparation of pLDSG from pCVSVL2 (ATCC No. 39813) and a t-PA encoding cDNA has been described in detail as has cotransformation with, and amplification of, pLDSG in CHO cells.. Kaufman et al., Mol. and Cell. Bio. 5(7): 1750-1759 (1985).

Plasmid pWGSM is identical to pLDSG except that the cDNA insert encodes Val-245 human t-PA. pWSGM may be constructed using cDNA from plasmid J205 (ATCC No. 39568) or pIVPA/I (ATCC No. 39891). Throughout this disclosure pWGSM and pLDSG may be used interchangeably, although as indicated previously, the former vector will produce Val-245 proteins and the latter Met-245 proteins.

pIVPA/l (ATCC No. 39891) is a baculoviral transplacement vector containing a t-PA-encoding cDNA. pIVPA/l and mutagenized derivatives thereof are used to insert a desired cDNA into a baculoviral genome such that the cDNA will be under the transcriptional control of the baculoviral polyhedrin promoter.

### Heteroduplex Mutagenesis

The mutagenesis via heteroduplexed DNA of specfic areas in the t-PA expression plasmid, pSVPA4, involves the following steps:

### Preparation of ampicillin sensitive pSVPA4 DNA

1. Plasmid pSVPA4 (15 ug) was linearized with Pvul to completion. This mixture was extracted with phenol/chloroform and the DNA was precipitated using two volumes of ethanol with 0.1 M NaCl present.
2. The DNA was resuspended in a 21 ul of water, 1 ul dNTB solution (containing 2mM dATP, dGTP, dTTP, dCTP), 2.5 ul 10X nick translation buffer (0.5M Tris-CI pH 7.5, 0.1 M MgSO₄, 10 mM DTT, 500 ug/ml) and 0.5 ul (2 units) DNA polymerase 1-Large Fragment (New England Biolabs). This mixture was incubated at room temperature for thirty minutes and then phenol/chlorform extracted followed by ethanol precipitation as described above.
3. The precipitated DNA was resuspended to 0.2 ug/ul by the addition of 75 ul water.

### Preparation of ampicillin resistant pSVPA4 DNA

1. Plasmid pSVPA4 (15 ug) was digested with Sac I which cuts this plasmid twice within the t-PA encoding sequence to produce two restriction fragments, a 1.4 kbp t-PA encoding restriction fragment plus the parent vector. Following restriction digestion 1 ul (28 units) of calf intestine alkaline phospatase (Boehringer Mannheim) was added then incubated at 37°C for five minutes. The two bands were separated by loading this mixture onto a 0.7% agarose gel. The parent vector restriction fragment was excised from the gel and extracted by adsorption to silica dioxide at 4 °C, which was folowed by elution in 50 mM Tris/1mM EDTA at 37°C for thirty minutes. The eluted DNA was adjusted to a final concentration of 0.2 ug/ul.

### Heteroduplex Annealing

1. Mix 6 ul (1.2 ug) of ampicillin sensitive pSVPA4 DNA with 6 ul (1.2 ug) ampicillin resistant pSVPA4 DNA.
2. Add an equal volume (12 ul) of 0.4 M NaOH. Incubate at room temperature for ten minutes.
3. Slowly add 4.5 volumes (108 ul) of 0.1 M Tris-CI pH 7.5/20 mM HCI.
4. 50 picomoles (5 ul) of phosphorylated mutagenic oligonucleotide was added to 45 ul of heteroduplex mixture.
5. This mixture was incubated at 68°C for two hours then slowly cooled to room temperature.

### Mutagenesis

1. Each mutagenesis reaction was adjusted to the following concentrations by the addition of 7 ul to the heteroduplex mixtures, 2mM MgCl/0.2 mM ATP/60uM dATP, dTTP,dGTP,dCTP/4 mM DTT/40. units/ml Klenow fragment of E. coli DNA polymerase I (B.R.L.), 2000 units/ml T4 DNA ligase (N.E.B.). This mixture was incubated at room temperature for 2 hours.
2. The reaction was then phenol/chloroform extracted which was followed by ethanol precipitation. The precipitated DNA was resuspended in 12 ul 50mM Tris-Cl/1mM EDTA. 4ul of this was used to transform competent HB101 bacteria.
3. Ampicillin resistant colonies were screened with 1x10⁶ cpm/ml of a ³²P-labeled screening oligonucleotide in 5X SSC, 0.1% SDS, 5Xdenhardt's reagent, and 100 ug/ml denatured salmon sperm DNA.
4. The filters were washed with 5X SSC, 0.1% SDS at a temperature 5° below the calculated melting temperature of the oligonucleotide probe.
5. DNA was prepared from positively hybridizing clones and analyzed initially by digestion with different restriction enzymes and agarose gel electrophoresis. DNA was transferred to nitrocellulose and filters were prepared and hybridized to the screening probes in order to ensure the mutagenic oligonucleotide was introduced in to the correct fragment.
6. DNA was then retransformed into E. coli and ampicillin resistant colonies were screened for hybridization to the screening oligonucleotide.
7. Final mutations were confirmed by DNA sequencing (Sanger).

### Preparation of Mutagenized cDNAs: MI3 method

The following schematic restriction map illustrates a cDNA encoding human t-PA (above) with cleavage sites indicated for specific endonucleases (indicated below): 
ATG R1 R2 R3
Sac Bgl Nar Eco Eco Sac Apa Xma
I II I RI RI I I I
(SmaI)
 The initiation codon, ATG, and the cDNA regions encoding (a), R¹, R² and R³ are indicated. Thus, mutagenesis at the N-terminus may be effected using the Sacl fragment or the BglII/NarI fragment, for example. Mutagenesis at Arg-275 and/or at R¹ and/or R² may be effected using, e.g., the Sacl fragment or BglII/SacI fragment. Mutagenesis at R³ may be effected using, an EcoRI/Xmal or EcoRI/Apal fragment. The choice of restriction fragment may be determined based on the convenience of using particular vectors for mutagenesis and/or for expression vector construction.

Generally, the cDNA restriction fragment to be mutagenized may be excised from the full-length cDNA present, e.g., in pWGSM, pIVPA/I or pSVPA4, using the indicated endonuclease enzyme(s) and then mutagenized, e.g. with the oligonucleotides shown in Table 7 or other oligonucleotides designed for the desired mutagenesis.
Exemplary mutagenized cDNA fragments which may thus be prepared are shown in Table 8, below.

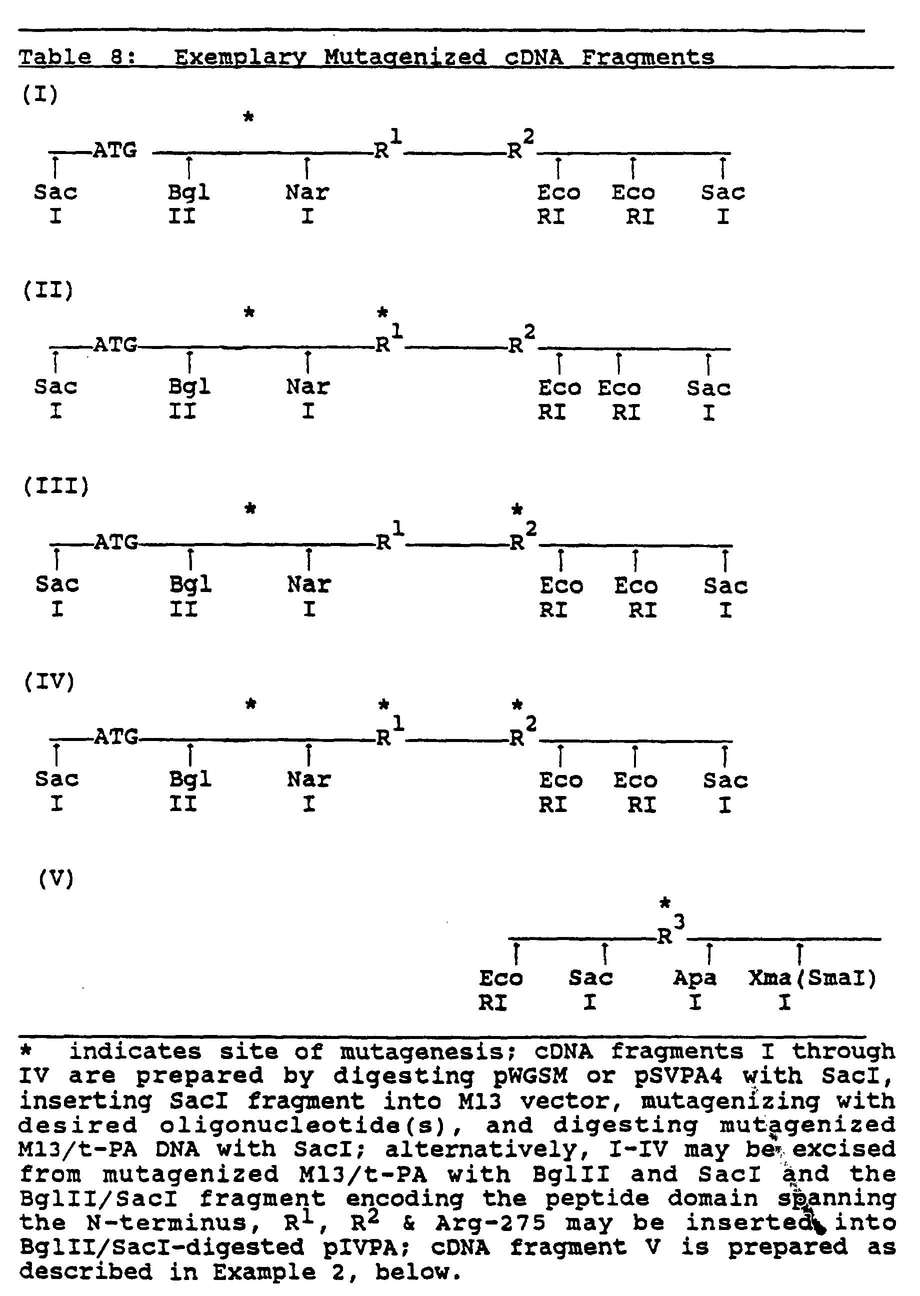

Following mutagenesis the fragment, with or without further mutagenesis, may then be excised from the MI3 vector and ligated back into an expression vector containing the full-length or partial cDNA previously cleaved with the same enzyme(s) as were used for excising the mutagenized fragment from the MI3 vector. By this method the full-length cDNA, mutagenized as desired, may be re-assembled using one or more mutagenized fragments as restriction fragment cassettes.

cDNAs encoding the following illustrative compounds (see Table, page 7 and Tables 2.0, 2.5 & 3) may be prepared from the mutagenized fragments of Table 8 as follows:

| Compound | Route | |
|---|---|---|
| D-6, D-1 D-3 | (a) | ligate mutagenized cDNA fragment I (prepared using oligonucleotides #8,10 or 12) into SacI-digested pSVPA4, or excise fragment I from mutagenized M13/t-PA as the BglII/SacI fragment and insert same into BglII/SacI-digested pIVPA/1. |
| | | |
| 2-1/N-21/Arg | (b) | ligate mutagenized cDNA fragment II (prepared using oligonucleotides #8,10 or 12) and then oligonucleotide #3) into SacI-digested pSVPA4, or excise fragment II from mutagenized M13/t-PA as the BglII/SacI fragment and insert same into BglII/SacI-digested pIVPA/l. |
| | | |
| 2-2/N-21/Arg | (c) | ligate mutagenized cDNA fragment III (prepared using oligonucleotides #8,10 or 12 and oligonucleotide #5) into SacI-digested pSVPA4 or excise fragment III from mutagenized M13/t-PA as the BglII/SacI fragment and insert same into BglII/SacI-digested pIVPA/l. |
| | | |
| 2-3/N-21/Arg | (d) | digest mutagenized pIVPA/l or pSVPA4 produced by Route (a) with EcoRI (partial digest) and XmaI (SmaI) or ApaI (total digest) to remove wild type R³ coding region, and ligate thereto mutagenized cDNA fragment V (prepared using oligonucleotide #7) as the EcoRI/ApaI or EcoRI/XmaI (SmaI) fragment. |
| | | |
| 2-4/N-21/Arg | (e) | digest mutagenized pIVPA or pSVPA prepared as in Route (c) with EcoRI (partial digest) and XmaI (SmaI) or ApaI (total digest) to remove wild type R³-coding region, and ligate thereto cDNA fragment V (prepared using oligonucleotide #7) as the EcoRI/ApaI or EcoRI/XmaI (SmaI) fragment. |
| | | |
| 2-5/N-21/Arg | (f) | digest mutagenized pIVPA or pSVPA4 prepared by Route (b) with EcoRI (partial digest) and XmaI (SmaI) or ApaI (total digest) and ligate thereto mutagenized cDNA fragment V (prepared using oligonucleotide #7) as the EcoRI/ApaI or EcoRI/XmaI (SmaI) fragment. |
| | | |
| 2-6/N-21/Arg | (g) | ligate mutagenized cDNA fragment IV (prepared using oligonucleotides #8,10 or 12 and oligonucleotides #3 and 5) into SacI-digested pSVPA4 or excise fragment IV from mutagenized M13/t-PA as the BglII/SacI fragment and ligate same into BglII/SacI-digested pIVPA/1. |
| | | |
| 2-7/N-21/Arg | (h) | ligate mutagenized cDNA fragment IV (prepared using oligonucleotides #8,10 or 12 and oligonucleotides #3 and 5) into SacI-digested pSVPA4 prepared by Routes (d), (e) or (f) or ligate fragment IV so produced as the BglII/SacI fragment into BglII/SacI-digested pIVPA produced by Route(s) (d), (e), of (f). |

Plasmids pIVPA or pSVPA4, in addition to utility as expression vectors, may also be used as a "depot" in the construction of cDNAs having any desired permutation of mutagenized sites. Thus, "pIVPA/Δ" or "pSVPA4/Δ ", mutagenized (via MI3 or heteroduplexing) plasmids containing a desired modification in the cDNA region encoding the N-terminal region may be digested with Narl (partial) and Xmal (Smal) (total) to remove the cDNA region encoding the protein domain spanning R¹, R² and R³. A second pIVPA or pSVPA4 plasmid mutagenized, if desired (via MI3 or heteroduplexing), at any combination of Arg-275, R¹, R² and R³-encoding regions may then be digested with Narl (total) and Xmal (Smal) (total) and the NarI/XmaI (Smal) fragment may then be identified, isolated and ligated into the Narl/Xmal (Smal) digested pIVPA/Δ or pSVPA4/Δ. Such use of the Narl/Xmal (Smal) restriction fragment cassette, for example, allows the construction of desired mutagenized cDNAs in pIVPA or pSVPA4. The mutagenized cDNA may then be transferred, e.g. as a BglII/XmaI restriction fragment cassette into BglII/Xmal-digested pWGSM for mammalian expression, if desired.

### EXAMPLES

### Example 1

### Preparation of Gln₁₁₇ Deletions Variants

### A. Preparation of Gln-117 truncated cDNA

cDNA molecules encoding the polypeptide sequence of compound 2-1/N-21/Arg was prepared using the oligonucleotide-directed mutagenesis method of Zoller and Smith. Specifically, the mutagenesis vector RF MI3/t-PA containing the t-PA gene was constructed from the mammalian t-PA expression plasmid pSVPA4. RF MI3/t-PA was constructed by first digesting pSVPA4 to completion with the restriction endonuclease Sacl. The approximately 1,436 base pair (bp) Sacl fragment encodes a large portion of the polypeptide sequence of t-PA and includes the nucleotide sequences encoding the consensus N-linked glycosylation sites encompassing asparagines 117,184, and 218. This 1,436 bp (hereinafter 1.4 kbp) fragment was purified by preparative agarose gel electrophoresis.

The Sac I fragment of the t-PA cDNA, obtained as a Sacl fragment, above, was ligated to a linearized double-stranded RF MI3mp18 DNA vector which had been previously digested with Sac I. The ligation mixture was used to transform transformation competent bacterial JM101 cells. MI3 plaques containing t-PA-derived DNA produced from transformed cells were identified and isolated by analytical DNA restriction analysis and/or plaque hybridization Radiolabeled oligonucleotides (∼17mers, of positive polarity) derived from within the Sacl restriction sites of the t-PA-encoding nucleotide sequence depicted in Table 1 were used as probes when filter hybridization was employed to detect viral plaques containing tPA DNA. All oligonucleotides were prepared by automated synthesis with an Applied Biosystems DNA synthesizer according to the manufacturer's instructions.

Several of the positive plaques detected by restriction or hybridization analysis were then further cloned by conventional plaque purification, Purified MI3/t-PA bacteriophage obtained from the plaque purification procedure was used to infect JM101 cells. These infected cells produce cytoplasmic double-stranded "RF" MI3/t-PA plasmid DNA. The infected cells also produce bacteriophage in the culture medium which contain single-stranded DNA complimentary to the 1.4 kbp SacI fragment of t-PA and to MI3 DNA. Single-stranded DNA was purified from the MI3/t-PA-containing phage isolated from the culture medium. This single-stranded MI3/t-PA DNA was used as a template in a mutagenesis reaction according to the method of Zoller and Smith using oligonucleotide #3 of Table 7: This mutagenesis event changes the Asn codon to a GIn codon at position 117 of the subsequently obtained coding strand of DNA by changing the DNA sequence from "AAC" to "CAG". Following the mutagenesis reaction, the DNA was transformed into the bacterial strain JM 101. To identify mutagenized cDNA's, the transformant plaques were screened by DNA hybridization using radiolabeled oligonucleotide #4 of Table 7. All exemplary oligonucleotides in Table 7 are of positive polarity, i.e., represent portions of a coding rather than non-coding strand of DNA. All hybridization positive plaques were further purified by subsequent secondary infections of JM 101 cells with MI3 phage containing mutagenized DNA.

RF MI3/t-PA plasmid DNA was purified from JM 101 cells infected with purified MI3 phage containing mutagenized t-PA cDNA. The RF MI3/t-PA plasmid thus obtained contains the Gln₁₁₇ mutagenized Sac I restriction fragment of t-PA DNA. This mutagenized restriction fragment can then be further mutagenized, again by the method of Zoller and Smith, but using the oligonucleotides described below. The oligonucleotides described below were designed to induce a deletion ("loop out") within the cDNA region encoding the N-terminal domain.

Deletion Mutagenesis 1: Oligonucleotide #8 of Table 7 induced a cDNA deletion encoding Cys-6 through Ser-50, inclusive. Following this second mutagenesis reaction the DNA is transformed into JM 101 cells. To identify mutagenized cDNAs, the transformant plaques were screened as above, but using radiolabeled oligonucleotide #9 of Table 7. Hybridization positive plaques can be further purified by subsequent secondary infections of JM 101 cells with MI3 phage containing the twice mutagenized t-PA cDNA. The cDNA prepared as described below which contains this mutagenized restriction fragment encodes compound 2-1/N-21/Arg in which Ile-5 is covalently bonded to Cys-51 by a peptide bond.

This mutagenized restriction fragment can then be ligated back into the mammalian expression vector pSVPA4 as a Sac I cassette by methods analogous to those described in Example #3B, or prepared for insertion into the insect cell expression vector pIVPA/I (ATCC No.39891) as a Bgl II/Sac I cassette derived from modified RF MI3/t-PA DNA.

### B. Preparation of Vectors Used for Expression of High Mannose Gln₁₁₇ Deletion Variants

The purified RF MI3/t-PA containing the modified and truncated t-PA cDNA, prepared as described above, can be digested with the restriction endonucleases Bglll and Sac I. The approximately 1.2 kbp BglII/Sac I restriction fragment was purified by conventional preparative gel electrophoresis. The BglII/Sac I fragment so obtained constitutes a mutagenized cassette which lacks a 5' and 3' portion of the DNA which encodes the amino and carboxy termini of the translated protein.

Insect expression vector pIVPA/I (ATCC No. 39891) contains a wild type tPA cDNA insert operatively linked to a polyhedrin promoter together with baculovirus flanking DNA sequences. pIVPA/l was digested with BglII and Sac I thereby excising a t-PA coding region spanning the N-terminus and R' and R². The BglII/Sac I cassette containing the mutagenized, N-terminally modified t-PA cDNA fragments may each then be ligated to pIVPA/l expression vector DNA which had been previously purified following digestion with BglII and SacI. The resulting plasmid, pIVPA/Δ FBR; Gln₁₁₇ should contain the mutagenized cDNAs encoding compound 2-1/N-21/Arg, now operatively linked to the polyhedrin promoter. The nucleotide sequence of this mutagenized cDNA insert may be confirmed by supercoil sequencing with plasmid as substrate. See e.g, E.Y. Chen et al., 1985, DNA 4(2):165-170.

### B. Introduction of the Mutagenized cDNA into the Insect Virus

The pIVPA plasmid containing the mutagenized cDNA may be introduced into the insect virus by co-transfection with wild-type AcNPV in Spodoptera cells. 1 ug of purified Autographa californica NPV DNA and 10ug of the desired pIVPA DNA are introduced into Spodoptera cells growing on tissue culture dishes by a calcium phosphate transfection procedure (Potter, K.N. and Miller, L.K., J.Invertebr. Path. (1980), 36 431-432). The joint introduction of these DNAs into the cells results in a double recombination event between the pIVPA plasmid (containing the mutagenized cDNAs) and the viral DNA at the regions of homology between the two; that is, the polyhedrin gene region of the progeny virus from the recombination event contains the mutagenized cDNA insert from the pIVPA plasmid.

### Isolation of Virus Containing the Nucleotide Sequence Encoding the Proteins of this Invention

The progeny virus present in the media over the transfected cells are plaqued onto a fresh monolayer of cells at several different dilutions. Plaques are assayed, and the recombinants are picked based on the PIB-minus phenotype as follows: A virus which has lost its polyhedrin gene, as would a virus containing a mutagenized cDNA will not produce PIBs. Plaques that appear PIB deficient are selected, excised and amplified on fresh cells. The supernatant over these cells is then assayed for t-PA-type enzymatic activity. Positive assay results indicate that the glycoprotein is in fact being produced.

An alternative method of virus purification via the plaque lifting protocol differs slightly from the steps described above, and is described below. Plaque the progeny virus from transfection at suitable dilution onto cell culture dishes. Prepare a nitrocellulose replica of the cell monolayer and the virus plaques. Reserve the agarose overlay from the plate as the virus source after the results of the following steps are obtained.

Probe the nitrocellulose filter with radioactive DNA fragments representative of the gene being placed into the viral chromosome. Score positives as those containing the foreign gene. Remove the hybridized probe. Re-probe the filter with radioactive DNA representative of a portion of the viral chromosome removed by substitution with the foreign DNA. One would score positives as those which still have a polyhedrin gene.

Remove the hybridized probe. Re-probe the filter with a radioactive DNA fragment which will identify viral plaques regardless of the state of the polyhedrin gene. A suitable fragment may be the EcoRI I fragment. Score these as progeny virus. Select those plaques which are positive for the foreign gene DNA probe, negative for the polyhedrin gene probe, and positive for the viral DNA probe. These are strong candidates for the desired genotype.

### C. Production and Characterization of High Mannose Glycoprotein

Antibodies have been used to demostrate the presence of the variant proteins in the extracellular media of infected cells. Recombinant virus, prepared as above, is used to infect cells grown in the usual TC-100 (Gibco) nutrient salts solution but instead of the standard media supplement of 10% fetal calf serum, this is replaced with a 50% egg yolk enrichment (to 1% total volume) ( Scott Biologicals). Previous experiments had demonstrated a more intact protein under these conditions. The supernatant from the infected cells is fractionated on an affinity column bearing an attached monoclonal antibody to natural human t-PA. Protein specifically retained by the column is eluted and assayed for t-PA enzymatic activity. A fixed amount of activity units of this and control t-PA preparations are separated on an acrylamide gel. This gel is then stained with a silver-based reagent to display the protein pattern. This reveals that the virus, upon infection of insect cells, leads to the extracellular production of a protein having t-PA type activity.

Radiolabeled protein is produced for further characterization by first incubating spodoptera frugiperda cells infected with the virus (m.o.i=1) for 48 hours. The culture plates are then rinsed with methionine-deficient media. Methionine-deficient media supplemented with ³⁵S-methione is then added to the culture plates. The cell cultures are incubated for 4 hours. The supernatant containing the radiolabeled glycoprotein may be analyzed by SDS-PAGE (7.5%) against wild type (i.e. full-length fully glycosylated) t-PA analogously produced in insect cells and against mammalian t-PA produced e.g. by the method of R. Kaufman et al., Mol. Cell. Biol. 5(7):1750(1985)., but in the presence of tunicamycin (non-glycosylated). The partially glycosylated truncated proteins produced in Example 1 should have an increased gel mobility relative to the fully-glycosylated analog and to the non-glycosylated full-length analog.

### EXAMPLE 2

### PREPARATION OF OTHER PROTEINS OF THIS INVENTION.

### A. Preparation of other cDNA's

The mutagenesis methods of Example 1 can be used with other conventionally prepared synthetic oligonucleotides which modify the original t-PA DNA sequence to produce proteins modified at the N-terminal region and/or optionally modified at N-linked glycosylation sites and/or at Arg-275 with the appropriate codon change(s) described previously. See, e.g. "Preparation of Mutagenized cDNAs: MI3 Method" and Routes (a)-(h), supra.

For example. cDNA encoding Compounds D-6, D-1 and D-3 may be prepared using the SacI restriction fragment in MI3/t-PA and mutagenizing with oligonucleotides #8, 10 and 12 respectively, but not with oligonucleotide #3. Arg-275 may be deleted or replaced, eg with Thr, using oligo's 14 or 15, respectively. Vector construction, transfection and expression may be carried out as in Example 1 for insect cells or as described below in Example 3 for mammalian cells.

Single-stranded DNA generated from the MI3 mutagenesis vector (RF MI3/t-PA), prepared as in Example 1, can also be used as a template to mutagenize, in a site specific manner, at Arg-275 and/or at glycosylation site(s) R¹ or R² or both. The region encoding the consensus tripeptide which encompasses Asn₂₁₈ may be similarly mutagenized. To prepare multiple modifications of the protein at these sites an iterative process may be used. For example, following the identification and the purification of MI3 phage containing a modified R¹ site, single-stranded DNA containing this modified site can be purified from phage and used as template to initiate a second round of mutagenesis within the R² site and/or at Arg-275. This process can be repeated until all desired modifications are obtained. Thus, cDNA encoding Compounds 2-2-2/N-21/Arg may be prepared by the method of Example 1 but substituting mutagenesis oligonucleotide #5 for oligonucleotide #3 and screening oligonucleotide #6 for oligonucleotide #4. cDNA encoding Compounds 2-6/N-21/Arg may be prepared by twice mutagenizing the Sacl fragment as described in Example 1 and addition mutagenizing and screening with oligonucleotides #5 and #6. Vector construction, transfection and expression are carried out as in Example 1 for insect cells or as described below for mammalian cells. See Routes (a)-(h), supra.

The RF MI3/t-PA mutagenesis vector does not contain DNA sequence encoding R³, the N-linked glycosylation site of t-PA most proximal to the carboxy-terminus of the protein. Therefore in order to make DNA modifications at that site, a new MI3/t-PA mutagenesis RF vector called MI3/t-PA:RI-Xma I was made. This vector was constructed by digesting the MI3 vector MI3mp11 to completion with EcoRI and Xma I. The RI/XmaI digested MI3 vector was ligated to a purified EcoRI/Xma I t-PA restriction fragment (approximately 439bp. hereinafter 0.4kbp) encoding a polypeptide region encompassing glycosylation site R³. This 0.4kbp restriction fragment was purified following digestion of the plasmid pWGSM with EcoRI and Xma I. The mammalian expression plasmid pWGSM, encoding the t-PA gene, is identical within the 439bp EcoRI/Xma I fragment to the plasmid pLDSG described by Kaufman et al., Mol. Cell Biol. 5:1750-1759 (1985).

The ligation mixture was used to transform competent bacterial JM 101 cells. Several plaques were picked and analyzed for the presence of the 0.4kbp t-PA EcoRI/XmaI fragment by standard DNA restriction fragment analysis.
Double-stranded RF MI3 DNA was purified from cells containing the 0.4kbp t-PA fragment. This DNA was designated RF MI3/t-PA:RI-Xma I mutagenesis vector. As previously indicated in Example 1A this vector, when transformed into competent JM101 cells, can be used to make MI3/t-PA:RI-Xmal phage from which single-stranded MI3/t-PA:RI-Xmal DNA can be purified. This single-stranded DNA can be used as template in the site-directed mutagenesis reaction to modify the t-PA DNA at the N-linked glycosylation site R³.

Modified R³ coding sequences can be used to replace the wild-type R³ sequences present in either modified pIVPA/l as prepared in Example 1 (truncated and/or modified at R¹ and/or R²) or wild-type pIVPA/l plasmid DNA. This can be accomplished by first performing a total Sac l/Apa I digestion of the R³ modified MI3/t-PA:RI/Xmal mutagenesis plasmid vector, and isolating the R³ modified 165 base pair t-PA restriction fragment so produced. The insect expression vector pIVPA/l or pIVPA/l plasmid DNA modified, e.g. as in Example 1, can similarly be totally digested with Sac I and Apa I to excise the 165 bp wild-type t-PA restriction fragment encoding the unmodified R³ site. Ligation of the purified insect expression vector lacking the 165 bp fragment to the modified R³ 165 bp fragment produces a new insect expression vector. Expression of the vector produces a truncated protein modified at the R³ site, as well as at any or all of the other consensus N-linked glycosylation sites present in natural t-PA and/or at Arg-275.

The pIVPA plasmid containing the modified cDNA may also be used to generate the BglII/ApaI fragment of the modified t-PA cDNA which spans the deletion region in the N-terminal domain as well as the region encoding R¹, R² and R³ or the Narl/Xmal fragment which spans R¹, R² and R³. Either of those fragments may be inserted into mammalian expression vectors such as pSVPA4 or pWGSM as described in Example 3.

### EXAMPLE 3

### PREPARATION OF COMPOUNDS D-6, D-1 and D-3 IN MAMMALIAN CELLS

### A. Preparation of cDNA.

cDNA molecules encoding the polypeptide sequences of compounds D-6, D-1 and D-3 were prepared using mutagenesis oligonucleotides #8, 10, and 12, respectively, and the Sacl fragment of the t-PA cDNA as template by the MI3 method of Example 1 or heteroduplex mutagenesis (Moranaga Heteroduplex Mutagenesis protocol; both, supra). Mutants selected by DNA hybridization using screening oligonucleotides 9, 11 and 13 respectively were confirmed by DNA sequence analysis to be correct in the modified DNA sequence.

### B. Modified t-PA Vector Preparation

Each modified cOMA prepared in Example 1A (Δ, Gln₁₁₇) or 3A (Δ) was first removed from the MI3 mutagenesis vector RF MI3/t-PA by total digestion of the vector with Sacl. The approximately 1.4kbp restriction fragment of each mutagenized cDNA was purified by gel electrophoresis and then ligated into pSVPA4 as follows. First, pSVPA4 was digested with Sacl to remove the wild type t-PA 1.4kbp restriction fragment. The remaining portion of the Sacl digested pSVPA4 was then ligated to the 1.4kbp restriction fragment of the mutagenized cDNA. This ligation event can produce two orientations of the inserted fragment. The appropriate orientation in each case may be identified using EcoRI and Pvull as the enzymes in conventional analytical restriction enzyme analysis. This replacement allows the Sac I fragment to be used as a cassette fragment between the RF MI3/t-PA mutagenesis vector and the pSVPA4 mammalian expression vector. Modified MI3 Sacl fragments (truncated and optionally modified at R¹ and/or R²) may be inserted into Sacl-digested pSVPA4 DNA which has been previously, or is subsequently, modified at R³ if desired. Alternatively, DNA previously modified at R¹, R² and/or R³ can be excised from vectors such as pIVPA or pSVPA4 as a Narl/Apal or Narl/Xmal fragment. The fragment so obtained may then be inserted into vectors such as pSVPA4 or pWGSM previously digested with Narl (partial) and Apal or Xmal (total). By this method any combination of N-terminal deletion and/or substitution and/or glycosylation site mutagenesis and/or Arg-275 mutagenesis may be achieved.

### C. Transfection of COS (SV40 transformed African Green Monkey Kidney) Cells

COS-I cells (ATCC CRL 1650) were transfectd by the method of Lopata, M.A. et al., Nucl. Acids Res. 12:5707-5717 (1984) with the vectors prepared in Example 3B, i.e., modified pSVPA4. Serum containing medium was replaced with serum-free medium 24 hours after the transfection and conditioned medium was assayed for both the presence of plasminogen activating activity, using the chromogenic substrate S-2251, or the presence of t-PA antigen by an ELISA assay, 48 and 72 hours post-transfection.

### D. Viral propagation in CVI (African Green Monkey Kidney) cells.

Modified complex carbohydrate protein can be produced by infecting CVI cells (ATCC CCL 70) with SV40 viral stocks propagated as described by Gething and Sambrook (Nature 293:620-625, 1981). This has been carried out by first totally digesting modified pSVPA4 with the restriction endonuclease BamHI to remove the bacterial shuttle vector pXf3 from the SV40 viral DNA. Before transfecting this DNA into CVI cells, along with the helper virus SV40-rINS-pBR322 DNA (described below), the Bam HI linearized SV40/t-PA DNA is circularized by ligation at dilute DNA concentrations (1 ug/ml). This process was repeated with the insulin containing SV40 vector SV40-rINS-pBR322 (Horowitz, M. et al., 1982, Eukaryotic Viral Vectors, pp. 47-53, Cold Spring Harbor Laboratory). The bacterial shuttle vector pBR322 in SV40-rINS-pBR322 was removed by a total EcoRI digestion. The linearized insulin/SV40 viral DNA was then circularized by ligation at a DNA concentration of 1 ug/ml. It is necessary to transfect CV-I cells with circular ligated pSVPA4 and SV40-rINS DNAs, at equimolar amounts in order to generate viral stocks. SV40-rINS is used to provide "late" SV40 proteins while pSVPA4 provides the "early" SV40 proteins necessary for virus DNA production while also encoding the proteins of this invention: Consequently when cells are transfected with both these DNA's as described by Gething and Sambrook, SV40 virus is produced which contains DNA from either viral vectors. Subsequent infection of CVI cells with amplified virus has produced protein with t-PA-type activity which can be assayed 72 hours post-infection as described in Example 3C.

### Example 4

### Preparation of Other Proteins

cDNAs encoding various proteins of this invention have been prepared by the methods of Examples 1, 2 and 3. The Bgl II/Xmal restriction fragment cassette may then be excised from either the pIVPA or pSVPA4 vector containing the cDNA encoding the truncated protein with or without modification at one or more glycosylation sites. The excised BglII/XmaI fragment may then be ligated into Bgl II/Xmal-cut pSVPA4 or pWGSM for introduction into mammalian cells. Expression of such cDNAs in mammalian host cells, e.g. by the method of Example 3 or by the method of Kaufman et al., supra, (CHO host cells) or by the method of Howley et al., U.S. Patent No. 4,419,446 (1983) (BPV expression systems) yields the corresponding mammalian-derived truncated proteins. Thus, cDNA encoding compounds 2-1/N-21/Arg (Δ FBR, Gln₁₁₇) was prepared and inserted into pSVPA4 as described above. Similarly, cDNAs encoding Compounds D-1 (Δ FBR) and D-3 (Δ EGF/FBR) were prepared by MI3 mutagenesis, as described above. and inserted as the Sacl fragment into Sacl-digested pSVPA4. cDNA encoding Compound D-6 (Δ EGF) was prepared by the heteroduplex method, described above, using pSVPA4 as template and mutagenesis oligonucleotide #12, and screening with oligonucleotide #13.

To prepare the cDNAs encoding the proteins for amplification and expression in mammalian cells, cDNA contained in pSVPA4 or pIVPA is excised as a BglII/XmaI fragment and ligated into purified, BglII/Xmal-digested pWGSM. In each case the resulting pWGSM vector is introduced into CHO cells and amplified by the method of Kaufman, supra. The transformed and amplified CHO cells produce compounds D-6, D-1, D-3 and 2-1/N-21/Arg respectively. which were detected in the culture medium by human t-PA specific antibodies. The compounds may then be recovered and purified by immunoaffinity chromatography.

### Example 5

Example 4 may be repeated using cDNA encoding the proteins modified within the N-terminus and/or at R-275 with or without modification at R¹, R², and/or R³ to produce the desired protein in CHO cells. Mutagenized cDNAs may be prepared as described above. Thus, cDNAs encoding Compound 2-7/N-21/Arg are prepared in pIVPA as described in Example 2. The cDNAs may then be excised as the BglII/XmaI fragment and ligated into purified, BglII/XmaI-digested pWGSM, and the resultant vector transformed and amplified in CHO cells as in Example 4 to produce compound 2-7,N-21/Arg.

## Claims (Claims for the following Contracting State(s): BE, CH, LI, DE, FR, IT, NL, SE)

1. A thrombolytic protein having tissue plasminogen activator-type activity and having an improved fibrinolytic profile relative to nature human t-PA **characterized by** a peptide sequence of human t-PA that retains both kringle regions, wherein at least one of the consensus N-linked glycosylation sites is modified to other than a consensus N-linked glycosylation site and wherein
(a) one or more amino acids are deleted within the region Val-4 to Val-72; or
(b) one or more amino acids are replaced within the region Arg-23 to Val-72; or
(c) features (a) and (b) are combined; or
(d) the amino acids Cys-6 through Cys-51 are deleted; or
(e) the amino acids Cys-51 through Asp-87 are deleted; or
(f) the amino acids Cys-6 through Ile-86 are deleted.

2. The thrombolytic protein of claim 1(f), wherein the peptide sequence Asn-Ser-Ser spanning positions 117-119 in the peptide sequence of human t-PA is modified to other than a consensus N-linked glycosylation site.

3. The thrombolytic protein of claim 2, wherein Asn-117 is replaced with a different amino acid.

4. The thrombolytic protein of claim 3, wherein the N-linked glycosylation site at position 117-119 is modified such that Asn-117 is replaced with Gln, said thrombolytic protein being glycosylated at at least one unmodified N-linked glycosylation site.

5. The thrombolytic protein of any one of claims 1 through 4, wherein the amino acid at position 245 of the peptide sequence is Met or Val.

6. The thrombolytic protein of claim 5, wherein Arg-275 is deleted or is replaced by a different amino acid.

7. The thrombolytic protein of claim 1(f), wherein all three N-linked glycosylation sites which are glycosylated in human t-PA are modified to other than a consensus N-linked glycosylation site.

8. The thrombolytic protein of claim 7, wherein each of the Asn's at position 117, 184 and 448 are replaced with independently selected replacement amino acids.

9. The thrombolytic protein of claim 8, wherein the N-linked glycosylation sites at positions 117-119, 184-186 and 448-450 are modified such that Asn-117, Asn-184 and Asn-448 are replaced with Gln.

10. The thrombolytic protein of any one of claims 7 through 9, wherein the amino acid at position 245 of the peptide sequence is Met or Val.

11. The thrombolytic protein of claim 10, wherein Arg-275 is deleted or is replaced by a different amino acid.

12. A DNA molecule encoding a protein of any one of claims 1 to 11.

13. A thrombolytic protein having tissue plasminogen activator-type activity produced by expression of a DNA molecule of claim 12 in a host cell selected from the group consisting of mammalian, yeast, insect, fungal and bacterial cells.

14. A therapeutic composition for the treatment of thrombotic conditions which comprises an effective amount of a protein of any one of claims 1 to 11 in admixture with a pharmaceutically acceptable carrier.

## Claims (Claims for the following Contracting State(s): AT)

1. A process for the preparation of a thrombolytic protein having tissue plasminogen activator-type activity and having an improved fibrinolytic profile relative to nature human t-PA **characterized by** a peptide sequence of human t-PA that retains both kringle regions, said process comprising modifying at least one of the consensus N-linked glycosylation sites to other than a consensus N-linked glycosylation site and
(a) deleting one or more amino acids within the region Val-4 to Val-72; or
(b) replacing one or more amino acids within the region Arg-23 to Val-72; or
(c) combining features (a) and (b); or
(d) deleting the amino acids Cys-6 through Cys-51; or
(e) deleting the amino acids Cys-51 through Asp-87; or
(f) deleting the amino acids Cys-6 through Ile-86.

2. The process of claim 1(f), wherein the peptide sequence Asn-Ser-Ser spanning positions 117-119 in the peptide sequence of human t-PA is modified to other than a consensus N-linked glycosylation site.

3. The process of claim 2, wherein Asn-117 is replaced with a different amino acid.

4. The process of claim 3, wherein the N-linked glycosylation site at position 117-119 is modified such that Asn-117 is replaced with Gln, said thrombolytic protein being glycosylated at at least one unmodified N-linked glycosylation site.

5. The process of any one of claims 1 through 4, wherein the amino acid at position 245 of the peptide sequence is Met or Val.

6. The process of claim 5, wherein Arg-275 is deleted or is replaced by a different amino acid.

7. The process of claim 1(f), wherein all three N-linked glycosylation sites which are glycosylated in human t-PA are modified to other than a consensus N-linked glycosylation site.

8. The process of claim 7, wherein each of the Asn's at position 117, 184 and 448 are replaced with independently selected replacement amino acids.

9. The process of claim 8, wherein the N-linked glycosylation sites at positions 117-119, 184-186 and 448-450 are modified such that Asn-117, Asn-184 and Asn-448 are replaced with Gln.

10. The process of any one of claims 7 through 9, wherein the amino acid at position 245 of the peptide sequence is Met or Val.

11. The process of claim 10, wherein Arg-275 is deleted or is replaced by a different amino acid.

12. A process for the preparation of a DNA molecule encoding a protein of any one of claims 1 to 11 which comprises conventional site-directed mutagenesis of DNA sequences encoding t-PA or analogs or variants thereof.

13. A process for the preparation of a thrombolytic protein having tissue plasminogen activator-type activity which comprises expressing a DNA molecule obtained according to claim 12 in a host cell selected from the group consisting of mammalian, yeast, insect, fungal and bacterial cells.

## Patentansprüche (Patentansprüche für folgende(n) Vertragsstaat(en): BE, CH, LI, DE, FR, IT, NL, SE)

1. Thrombolytisches Protein mit Gewebe-Plasminogenaktivator-artiger Aktivität und mit verbessertem fibrinolytischem Profil verglichen mit natürlichem menschlichem t-PA, **gekennzeichnet durch** eine Peptidsequenz des menschlichen t-PA, welche beide Kringelregionen enthält, wobei mindestens eine der Konsensus-N-Glycosylierungsstellen zu etwas anderem modifiziert ist als zu einer Konsensus-N-Glycosylierungsstelle und wobei
(a) eine oder mehrere Aminosäuren aus der Region Val-4 bis Val-72 entfernt sind; oder
(b) eine oder mehrere Aminosäuren in der Region Arg-23 bis Val-72 ersetzt sind; oder
(c) die Merkmale (a) und (b) kombiniert sind; oder
(d) die Aminosäuren Cys-6 bis Cys-51 entfernt sind; oder
(e) die Aminosäuren Cys-51 bis Asp-87 entfernt sind; oder
(f) die Aminosäuren Cys-6 bis lle-86 entfernt sind.

2. Thrombolytisches Protein nach Anspruch 1(f), wobei die Peptidsequenz Asn-Ser-Ser, welche die Positionen 117 bis 119 in der Peptidsequenz des menschlichen t-PA umfaßt, zu etwas anderem als zu einer Konsensus-N-Glycosylierungsstelle modifiziert ist.

3. Thrombolytisches Protein nach Anspruch 2, wobei Asn-117 durch eine andere Aminosäure ersetzt ist.

4. Thrombolytisches Protein nach Anspruch 3, wobei die N-Glycosylierungsstelle an den Positionen 117 bis 119 so modifiziert ist, dass Asn-117 durch Gln ersetzt ist, wobei das thrombolytische Protein an mindestens einer nicht-modifizierten N-Glycosylierungsstelle glycosyliert ist.

5. Thrombolytisches Protein nach einem der Ansprüche 1 bis 4, wobei die Aminosäure an Position 245 der Peptidsequenz Met oder Val ist.

6. Thrombolytisches Protein nach Anspruch 5, wobei Arg-275 entfernt oder durch eine andere Aminosäure ersetzt ist.

7. Thrombolytisches Protein nach Anspruch 1(f), wobei alle drei N-Glycosylierungsstellen, welche im menschlichen t-PA glycosyliert sind, zu etwas anderem als zu einer Konsensus-N-Glycosylierungsstelle modifiziert sind.

8. Thrombolytisches Protein nach Anspruch 7, wobei jedes der Asn's an Position 117, 184 und 448 durch unabhängig ausgewählte Ersatzaminosäuren ersetzt ist.

9. Thrombolytisches Protein nach Anspruch 8, wobei die N-Glycosylierungsstellen an Positionen 117 bis 119, 184 bis 186 und 448 bis 450 so modifiziert sind, dass Asn-117, Asn-184 und Asn-448 durch Gln ersetzt sind.

10. Thrombolytisches Protein nach einem der Ansprüche 7 bis 9, wobei die Aminosäure an Position 245 der Peptidsequenz Met oder Val ist.

11. Thrombolytisches Protein nach Anspruch 10, wobei Arg-275 entfernt ist oder durch eine andere Aminosäure ersetzt ist.

12. DNA-Molekül, welches ein Protein nach einem der Ansprüche 1 bis 11 codiert.

13. Thrombolytisches Protein mit Gewebe-Plasminogenaktivator-artiger Aktivität, hergestellt durch Expression eines DNA-Moleküls nach Anspruch 12 in einer Wirtszelle ausgewählt aus der Gruppe bestehend aus Säuger-, Hefe-, Insekten-, Pilz- und Bakterienzellen.

14. Therapeutische Zusammensetzung zur Behandlung von thrombotischen Zuständen, welche eine wirksame Menge eines Proteins nach einem der Ansprüche 1 bis 11 im Gemisch mit einem pharmazeutisch verträglichen Träger umfaßt.

## Patentansprüche (Patentansprüche für folgende(n) Vertragsstaat(en): AT)

1. Verfahren zur Herstellung eines thrombolytischen Proteins mit Gewebe-Plasminogenaktivator-artiger Aktivität und mit verbessertem fibrinolytischem Profil verglichen mit natürlichem menschlichem t-PA, **gekennzeichnet durch** eine Peptidsequenz des menschlichen t-PA, welche beide Kringelregionen enthält, wobei das Verfahren das Modifizieren von mindestens einer der Konsensus-N-Glycosylierungsstellen zu etwas anderem als zu einer Konsensus-N-Glycosylierungsstelle umfaßt, sowie
(a) das Entfernen einer oder mehrerer Aminosäuren aus der Region Val-4 bis Val-72; oder
(b) das Ersetzen einer oder mehrerer Aminosäuren in der Region Arg-23 bis Val-72; oder
(c) das Kombinieren der Schritte(a) und (b); oder
(d) das Entfernen der Aminosäuren Cys-6 bis Cys-51; oder
(e) das Entfernen der Aminosäuren Cys-51 bis Asp-87; oder
(f) das Entfernen der Aminosäuren Cys-6 bis lle-86.

2. Verfahren nach Anspruch 1(f), wobei die Peptidsequenz Asn-Ser-Ser, welche die Positionen 117 bis 119 in der Peptidsequenz des menschlichen t-PA umfaßt, zu etwas anderem als zu einer Konsensus-N-Glycosylierungsstelle modifiziert ist.

3. Verfahren nach Anspruch 2, wobei Asn-117 ersetzt wird durch eine andere Aminosäure.

4. Verfahren nach Anspruch 3, wobei die N-Glycosylierungsstelle an den Positionen 117 bis 119 so modifiziert ist, dass Asn-117 durch Gln ersetzt ist, wobei das thrombolytische Protein an mindestens einer nicht-modifizierten N-Glycosylierungsstelle glycosyliert ist.

5. Verfahren nach einem der Ansprüche 1 bis 4, wobei die Aminosäure an Position 245 der Peptidsequenz Met oder Val ist.

6. Verfahren nach Anspruch 5, wobei Arg-275 entfernt oder durch eine andere Aminosäure ersetzt ist.

7. Verfahren nach Anspruch 1(f), wobei alle drei N-Glycosylierungsstellen, welche im menschlichen t-PA glycosyliert sind, zu etwas anderem als zu einer Konsensus-N-Glycosylierungsstelle modifiziert sind.

8. Verfahren nach Anspruch 7, wobei jedes der Asn's an Position 117, 184 und 448 durch unabhängig ausgewählte Ersatzaminosäuren ersetzt ist.

9. Verfahren nach Anspruch 8, wobei die N-Glycosylierungsstellen an Positionen 117 bis 119, 184 bis 186 und 448 bis 450 so modifiziert sind, dass Asn-117, Asn-184 und Asn-448 durch Gln ersetzt sind.

10. Verfahren nach einem der Ansprüche 7 bis 9, wobei die Aminosäure an Position 245 der Peptidsequenz Met oder Val ist.

11. Verfahren nach Anspruch 10, wobei Arg-275 entfernt ist oder durch eine andere Aminosäure ersetzt ist.

12. Verfahren zur Herstellung eines DNA-Moleküls, welches ein Protein gemäß einem der Ansprüche 1 bis 11 codiert, umfassend konventionelle ortsspezifische Mutagenese von DNA-Sequenzen, welche t-PA oder Analoge oder Varianten davon codieren.

13. Verfahren zur Herstellung eines thrombolytischen Proteins mit Gewebe-Plasminogenaktivator-artiger Aktivität, umfassend das Exprimieren eines DNA-Moleküls nach Anspruch 12 in einer Wirtszelle ausgewählt aus der Gruppe bestehend aus Säuger-, Hefe-, Insekten-, Pilz- und Baktierenzellen.

## Revendications (Revendications pour l'(les) Etat(s) contractant(s) suivant(s): BE, CH, LI, DE, FR, IT, NL, SE)

1. Une protéine thrombolytique ayant une activité de type activateur de plasminogène tissulaire et possédant un profil fibrinolytique amélioré par rapport au t-PA humain nature, **caractérisée par** une séquence peptidique du t-PA humain qui conserve les deux régions charnières, dans laquelle l'un au moins un des sites consensus de N-glycosylation est modifié autrement que en un site consensus de N-glycosylation, et dans laquelle
a) un ou plusieurs acides aminés sont délétés dans la région Val-4 à Val-72 ; ou
b) un ou plusieurs acides aminés sont remplacés dans la région Arg-23 à Val-72 ; ou
c) les caractéristiques a) et b) sont combinées ; ou
d) les acides aminés Cys-6 à Cys-51 sont délétés ; ou
e) les acides aminés Cys-51 à Asp-87 sont délétés ; ou
f) les acides aminés Cys-6 à Ile-86 sont délétés.

2. La protéine thrombolytique selon la revendication 1f), dans laquelle la séquence peptidique Asn-Ser-Ser couvrant les positions 117-119 dans la séquence peptidique du t-PA humain est modifiée autrement que en un site consensus de N-glycosylation.

3. La protéine thrombolytique selon la revendication 2, dans laquelle Asn-117 est remplacé par un acide aminé différent.

4. La protéine thrombolytique selon la revendication 3, dans laquelle le site de N-glycosylation en position 117-119 est modifié de sorte que Asn-117 est remplacé par Gln, ladite protéine thrombolytique étant glycosylée à au moins un site de N-glycosylation non modifié.

5. La protéine thrombolytique selon l'une quelconque des revendications 1 à 4, dans laquelle l'acide aminé en position 245 de la séquence peptidique est Met ou Val.

6. La protéine thrombolytique selon la revendication 5, dans laquelle Arg-275 est délétée ou est remplacée par un acide aminé différent.

7. La protéine thrombolytique selon la revendication 1f), dans laquelle tous les trois sites de N-glycosylation qui sont glycosylés dans le t-PA humain sont modifiés autrement que en un site consensus de N-glycosylation.

8. La protéine thrombolytique selon la revendication 7, dans laquelle chaque Asn en position 117, 184 et 448 est remplacée par un acide aminé de remplacement sélectionné de manière indépendante.

9. La protéine thrombolytique selon la revendication 8, dans laquelle les sites de N-glycosylation en positions 117-119, 184-186 et 448-450 sont modifiés de sorte que Asn-117, Asn-184 et Asn-448 sont remplacées par Gln.

10. La protéine thrombolytique selon l'une quelconque des revendications 7 à 9, dans laquelle l'acide aminé en position 245 de la séquence peptidique est Met ou Val.

11. La protéine thrombolytique selon la revendication 10, dans laquelle Arg-275 est délétée ou est remplacée par un acide aminé différent.

12. Une molécule d'ADN codant une protéine selon l'une quelconque des revendications 1 à 11.

13. Une protéine thrombolytique ayant une activité de type activateur de plasminogène tissulaire produite par expression d'une molécule d'ADN selon la revendication 12 dans une cellule hôte choisie parmi le groupe consistant en des cellules de mammifère, levure, insecte, champignon et bactérienne.

14. Une composition thérapeutique pour le traitement de conditions thrombotiques, qui comprend une quantité efficace d'une protéine selon l'une quelconque des revendications 1 à 11 en mélange avec un véhicule acceptable sur le plan pharmaceutique.

## Revendications (Revendications pour l'(les) Etat(s) contractant(s) suivant(s): AT)

1. Un procédé de préparation d'une protéine thrombolytique ayant une activité de type activateur de plasminogène tissulaire et possédant un profil fibrinolytique amélioré par rapport au t-PA humain nature, **caractérisée par** une séquence peptidique du t-PA humain qui conserve les deux régions charnières, ledit procédé comprenant la modification d'au moins un des sites consensus de N-glycosylation autrement que en un site consensus de N-glycosylation, et
a) la délétion d'un ou plusieurs acides aminés dans la région Val-4 à Val-72 ; ou
b) le remplacement d'un ou plusieurs acides aminés dans la région Arg-23 à Val-72 ; ou
c) la combinaison des caractéristiques a) et b); ou
d) la délétion des acides aminés Cys-6 à Cys-51; ou
e) la délétion des acides aminés Cys-51 à Asp-87; ou
f) la délétion des acides aminés Cys-6 à Ile-86.

2. Le procédé selon la revendication 1f), dans lequel la séquence peptidique Asn-Ser-Ser couvrant les positions 117-119 dans la séquence peptidique du t-PA humain est modifiée autrement que en un site consensus de N-glycosylation.

3. Le procédé selon la revendication 2, dans lequel Asn-117 est remplacé par un acide aminé différent.

4. Le procédé selon la revendication 3, dans lequel le site de N-glycosylation en position 117-119 est modifié de sorte que Asn-117 est remplacé par Gln, ladite protéine thrombolytique étant glycosylée à au moins un site de N-glycosylation non modifié.

5. Le procédé selon l'une quelconque des revendications 1 à 4, dans lequel l'acide aminé en position 245 de la séquence peptidique est Met ou Val.

6. Le procédé selon la revendication 5, dans lequel Arg-275 est délétée ou est remplacée par un acide aminé différent.

7. Le procédé selon la revendication 1f), dans lequel tous les trois sites de N-glycosylation qui sont glycosylés dans le t-PA humain sont modifiés autrement que en un site consensus de N-glycosylation.

8. Le procédé selon la revendication 7, dans lequel chaque Asn en position 117, 184 et 448 est remplacée par un acide aminé de remplacement sélectionné de manière indépendante.

9. Le procédé selon la revendication 8, dans lequel les sites de N-glycosylation en positions 117-119, 184-186 et 448-450 sont modifiés de sorte que Asn-117, Asn-184 et Asn-448 sont remplacées par Gln.

10. Le procédé selon l'une quelconque des revendications 7 à 9, dans lequel l'acide aminé en position 245 de la séquence peptidique est Met ou Val.

11. Le procédé selon la revendication 10, dans lequel Arg-275 est délétée ou est remplacée par un acide aminé différent.

12. Un procédé pour la préparation d'une molécule d'ADN codant une protéine selon l'une quelconque des revendications 1 à 11, comprenant la mutagénèse site-dirigée conventionnelle de séquences d'ADN codant t-PA ou des analogues ou variants de celui-ci.

13. Un procédé pour la préparation d'une protéine thrombolytique ayant une activité de type activateur de plasminogène tissulaire comprenant l'expression d'une molécule d'ADN obtenue selon la revendication 12 dans une cellule hôte choisie parmi le groupe consistant en des cellules de mammifère, levure, insecte, champignon et bactérienne.
